(19) Europäisches Patentamt
European Patent Office
Office européen des brevets

(11) EP 3 349 857 B1

(12) # FASCICULE DE BREVET EUROPEEN

(45) Date de publication et mention de la délivrance du brevet:
**28.07.2021 Bulletin 2021/30**

(21) Numéro de dépôt: **16766947.2**

(22) Date de dépôt: **16.09.2016**

(51) Int Cl.:
*A61Q 1/02* (2006.01) *A61K 8/04* (2006.01)
*B01J 13/10* (2006.01) *A61K 8/898* (2006.01)
*A61K 8/81* (2006.01) *A61K 8/37* (2006.01)
*A61K 8/34* (2006.01) *A61K 8/11* (2006.01)
*A61K 8/06* (2006.01) *A61Q 19/00* (2006.01)

(86) Numéro de dépôt international:
**PCT/EP2016/071934**

(87) Numéro de publication internationale:
**WO 2017/046305 (23.03.2017 Gazette 2017/12)**

(54) **DISPERSIONS STABLES DE GOUTTES COMPRENANT UN AGENT GELIFIANT**

STABILE DISPERSIONEN MIT TROPFEN MIT EINEM GELIERMITTEL

STABLE DISPERSIONS CONTAINING DROPS COMPRISING A GELLING AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.09.2015 FR 1558850**

(43) Date de publication de la demande:
**25.07.2018 Bulletin 2018/30**

(73) Titulaire: **Capsum**
**13013 Marseille (FR)**

(72) Inventeurs:
- **GOUTAYER, Mathieu**
**35400 Saint Malo (FR)**
- **PUJOL, Amélie**
**13004 Marseille (FR)**

(74) Mandataire: **Lavoix**
**2, place d'Estienne d'Orves**
**75441 Paris Cedex 09 (FR)**

(56) Documents cités:
**EP-A1- 1 808 479**
**WO-A1-2015/044084**
**WO-A1-2015/148892**
**US-A1- 2006 141 046**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 349 857 B1

## Description

La présente invention a pour objet des dispersions stables contenant des gouttes comprenant un agent gélifiant, ainsi que leurs utilisations dans le domaine cosmétique.

Il existe à ce jour des dispersions de gouttes d'une phase grasse dispersée dans une phase aqueuse, notamment décrites dans les demandes WO 2012/120043, FR 2 972 367 et FR 2 976 824. Ces dispersions sont notamment obtenues à l'aide d'un procédé microfluidique.

Les dispersions de ce type présentent généralement des résistances mécaniques faibles, ce qui peut conduire à des cisaillements ou des fragmentations des gouttes lors du transport des dispersions ou des produits cosmétiques les contenants. Il est également connu que les dispersions de ce type, lorsqu'elles sont obtenues à l'aide d'un procédé microfluidique, ne peuvent être commercialisées que dans des packagings requérant une atmosphère spécifique dénuée d'air (packaging dit « airless »), ce qui restreint leurs utilisations.

US 2006/0141046 décrit des particules avec un cœur interne comprenant un agent actif et une enveloppe externe formée d'une matrice comprenant des polymères anioniques et cationiques.

Il existe donc un besoin pour de nouvelles dispersions qui soient stables et qui présentent une résistance mécanique importante.

Par ailleurs, la simplification du procédé de préparation de telles dispersions demeure un objectif constant.

La présente invention a pour but de fournir une dispersion stable de gouttes dispersées dans une phase aqueuse continue.

Un autre but de l'invention est de fournir une dispersion de gouttes dispersées dans une phase aqueuse continue présentant des viscosités compatibles avec une manipulation aisée du produit obtenu.

Un autre but de l'invention est de fournir une dispersion de gouttes dispersées dans une phase aqueuse continue présentant des résistances mécaniques élevées, lui permettant notamment de résister à des cisaillements ou des fragmentations des gouttes lors du transport de ladite dispersion ou de produits cosmétiques la contenant.

Un autre but de l'invention consiste à fournir un procédé simplifié pour la préparation desdites dispersions.

Ainsi, la présente invention concerne une dispersion contenant une phase dispersée comprenant des gouttes et une phase aqueuse continue, de préférence sous forme de gel, dans laquelle les gouttes comprennent une phase grasse contenant au moins un agent gélifiant et une écorce, ladite écorce comprenant au moins un polymère anionique et au moins un polymère cationique, ladite dispersion étant différente d'une composition comprenant : 32,312% en poids d'eau, 0,8% en poids de phénoxyéthanol, 2% en poids de pentylèneglycol, 0,25% en poids de carbomère, 24,57% en poids de glycérine, 0,03% en poids d'EDTA disodique, 0,038% en poids d'hydroxyde de sodium, 12,8% en poids de polymère dimethicone/vinyl dimethicone crosspolymer, 27% en poids de diméthicone et 0,2% en poids d'amodiméthicone par rapport au poids total de ladite composition.

Dans le cadre de la présente invention, les dispersions susmentionnées peuvent être désignées indifféremment par le terme "émulsions".

Une goutte selon l'invention est composée d'un cœur, aussi appelé intérieur de la goutte, entouré d'une écorce, qui isole l'intérieur de la goutte de la phase continue de l'émulsion.

Selon un mode de réalisation, les dispersions selon l'invention ne comprennent pas de tensioactif. Elles se différencient donc des dispersions cosmétiques usuelles.

Les dispersions selon l'invention présentent un intérêt particulier en ce qui concerne la texture en se différenciant des émulsions « classiques » stabilisées par des tensioactifs. En effet, les dispersions selon l'invention se caractérisent par une texture unique, légère et volubile, procurant une application en deux temps. Plus particulièrement, les dispersions selon l'invention, voire les compositions les comprenant, s'étalent facilement sur la peau. Les premiers instants d'application sont très aqueux avec un effet cassant marqué. Puis, le ressenti évolue vers un voile huileux qui s'estompe pour laisser une peau légère et hydratée.

Cette texture est particulièrement avantageuse et surprenante pour l'homme du métier au vu de l'absence de tensioactifs dans ces émulsions.

Les dispersions selon l'invention présentent en outre un intérêt en ce qui concerne la texture et l'effet sensoriel en se différenciant des dispersions décrites dans WO/2012/120043. Plus particulièrement, l'application sur une matière kératinique, en particulier la peau, d'une dispersion selon l'invention conduit au moment de son étalement à un cisaillement des gouttes gélifiées. Ainsi, l'effet sensoriel procuré par cette application se traduit par le fait de sentir littéralement les gouttes gélifiées fondre sous l'effet de l'étalement et un effet huileux exacerbé.

La présente invention concerne également l'utilisation d'au moins un agent gélifiant de phase grasse pour améliorer la résistance mécanique des gouttes d'une dispersion selon l'invention.

Selon l'invention, le pH de la dispersion est typiquement compris de 5,0 à 7,0.

Selon un mode de réalisation, une dispersion selon l'invention est préparée par mise en œuvre d'un procédé « non-microfluidique », à savoir par simple émulsification, pour la préparation d'une dispersion selon l'invention, la taille des gouttes de la phase dispersée est inférieure à 500 μm, voire inférieure à 200 μm. Préférentiellement, la taille des

gouttes est comprise entre 0,5 μm à 50 μm, de préférence entre 1 μm et 20 μm.

Selon ce mode de réalisation, la présente invention permet ainsi de disposer de gouttes de taille réduite, notamment par rapport à des gouttes obtenues par un procédé microfluidique. Cette petite taille de gouttes va avoir un effet sur la texture. En effet, une composition selon l'invention, formée de gouttes finement dispersées, présente des qualités d'onctuosité améliorée.

Selon un autre mode de réalisation, une dispersion selon l'invention est préparée par mise en œuvre d'un procédé « microfluidique », notamment tel que décrit ci-après. Selon ce mode de réalisation, la taille des gouttes de la phase dispersée est supérieure à 500 μm, voire supérieure à 1000 μm. Préférentiellement, selon ce mode de réalisation, la taille des gouttes est comprise entre 500 et 3 000 μm, de préférence entre 1000 μm et 2000 μm.

A ce titre, il n'était pas évident que les émulsions comprenant de telles gouttes de taille supérieure à 500 μm soient stables.

Cette propriété intéressante en termes de stabilité cinétique est d'autant plus inattendue que l'écorce des gouttes, décrite en détail ci-après, est très fine. Ainsi, au moment de l'application sur une matière kératinique, aucune résistance attachée à la rupture de l'écorce n'est ressentie par l'utilisateur et aucun dépôt résiduel de ladite écorce n'est par ailleurs constaté. On parle ainsi d'écorce évanescente.

Les gouttes d'une dispersion selon l'invention, par la nature et les propriétés de leurs écorces, diffèrent donc de capsules solides, c'est-à-dire des capsules dotées d'une membrane solide, telle que par exemple celles décrites dans WO 2010/063937.

Dans le cadre de la présente invention, le terme "taille" désigne le diamètre, notamment le diamètre moyen, des gouttes.

### *Viscosité*

La viscosité des dispersions selon l'invention, voire des compositions les comprenant, peut varier de façon importante ce qui permet donc d'obtenir des textures variées.

Selon un mode de réalisation, une dispersion selon l'invention a une viscosité comprise de 1 mPa.s à 500 000 mPa.s, de préférence de 10 mPa.s à 300 000 mPa.s, mieux de 400 mPa.s à 100 000 mPa.s, et plus particulièrement de 1 000 mPa.s à 30 000 mPa.s, telle que mesurée à 25°C.

La viscosité est mesurée à température ambiante, par exemple T=25°C ± 2°C et à pression ambiante, par exemple 1013 mbar, par la méthode suivante.

On utilise un viscosimètre de type Brookfield, typiquement un viscosimètre numérique Brookfield RVDV-E (couple de torsion du ressort de 7187,0 dyne-cm), qui est un viscosimètre rotationnel à vitesse imposée muni d'un mobile (désigné par le terme anglais « Spindle »). Une vitesse est imposée au mobile en rotation et la mesure du couple exercé sur le mobile permet de déterminer la viscosité en connaissant les paramètres de géométrie/forme du mobile utilisé.

On utilise par exemple un mobile de taille No. 04 (référence Brookfield: RV4). Le taux de cisaillement correspondant à la mesure de la viscosité est défini par le mobile utilisé et la vitesse de rotation de celui-ci.

La mesure de viscosité est effectuée sur 1 minute à température ambiante (T=25°C ± 2°C). On place environ 150 g de solution dans un bécher de 250 ml de volume, ayant un diamètre d'environ 7 cm de façon à ce que la hauteur du volume occupée par les 150 g de solution soit suffisante pour arriver à la jauge marquée sur le mobile. Ensuite, on démarre le viscosimètre sur une vitesse de 10 tours/min et on attend que la valeur affichée sur l'écran soit stable. Cette mesure donne la viscosité du fluide testé, telle que mentionnée dans le cadre de la présente invention.

### *Phase continue aqueuse*

Comme indiqué précédemment, les dispersions selon l'invention comprennent une phase continue aqueuse, de préférence sous forme d'un gel, en particulier d'un gel présentant une viscosité adaptée pour suspendre les gouttes et contribuer ainsi au visuel attractif et inédit d'une dispersion selon l'invention.

Selon un mode de réalisation, la phase aqueuse a une viscosité comprise entre 400 mPa.s et 100 000 mPa.s, de préférence entre 800 mPa.s et 30 000 mPa.s, telle que mesurée à 25°C.

Cette viscosité est mesurée selon la méthode décrite ci-dessus.

La phase continue des dispersions comprend de l'eau.

Outre l'eau distillée ou déionisée, une eau convenant à l'invention peut être aussi une eau de source naturelle ou une eau florale.

Selon un mode de réalisation, le pourcentage massique d'eau de la phase continue aqueuse est d'au moins 30%, de préférence d'au moins 40%, en particulier d'au moins 50%, et mieux d'au moins 60%, notamment compris entre 70% et 98%, et préférentiellement compris entre 75% et 95%, par rapport à la masse totale de ladite phase continue.

La phase continue aqueuse de la dispersion selon l'invention peut en outre comprendre au moins une base. Elle peut comprendre une base unique ou un mélange de plusieurs bases différentes. La présence d'au moins une base

dans ladite phase continue aqueuse contribue notamment à rehausser la viscosité de cette dernière.

Selon un mode de réalisation, la base présente dans la phase aqueuse est une base minérale.

Selon un mode de réalisation, la base minérale est choisie dans le groupe constitué des hydroxydes des métaux alcalins et des hydroxydes des métaux alcalino-terreux.

De préférence, la base minérale est un hydroxyde de métaux alcalins, et notamment NaOH.

Selon un mode de réalisation, la base présente dans la phase aqueuse est une base organique. Parmi les bases organiques, on peut citer par exemple l'ammoniaque, la pyridine, la triéthanolamine, l'aminométhylpropanol, ou encore la triéthylamine.

Une dispersion selon l'invention peut comprendre de 0,01% à 10% en poids, de préférence de 0,01% à 5% en poids, et préférentiellement de 0,02% à 1% en poids de base, de préférence de base minérale, et notamment de NaOH, par rapport au poids total de ladite dispersion.

Selon un mode de réalisation, les dispersions selon l'invention ne comprennent pas de tensioactif.

Selon un autre mode de réalisation, la phase continue aqueuse peut en outre comprendre au moins un tensioactif.

L'agent tensioactif est avantageusement un tensioactif anionique, un tensioactif non ionique, un tensioactif cationique ou un mélange de ceux-ci. La masse moléculaire de l'agent tensioactif est comprise entre 150 g/mol et 10 000 g/mol, avantageusement entre 250 g/mol et 1 500 g/mol.

Dans le cas où le tensioactif est un tensioactif anionique, il est par exemple choisi parmi les alkylsulfates, les alkylsulfonates, les alkylarylsulfonates, les alkylphosphates alcalins, les dialkylsulfosuccinates, les sels d'alcalino-terreux d'acides gras saturés ou non. Ces tensioactifs présentent avantageusement au moins une chaîne hydrocarbonée hydrophobe présentant un nombre de carbones supérieur à 5, voire 10 et au moins un groupement anionique hydrophile, tel qu'un sulfate, un sulfonate ou un carboxylate lié à une extrémité de la chaîne hydrophobe.

Dans le cas où le tensioactif est un tensioactif cationique, il est par exemple choisi parmi les sels d'halogénures d'alkylpyridium ou d'alkylammonium comme le chlorure ou le bromure de n-éthyldodecylammonium, le chlorure ou le bromure de cétylammonium (CTAB). Ces tensioactifs présentent avantageusement au moins une chaîne hydrocarbonée hydrophobe présentant un nombre d'atomes de carbone supérieur à 5, voire 10 et au moins un groupement cationique hydrophile, tel qu'un cation d'ammonium quaternaire.

Dans le cas où le tensioactif est un tensioactif non ionique, il est par exemple choisi parmi des dérivés polyoxyéthylénés et/ou polyoxypropylénés des alcools gras, des acides gras, ou des alkylphénols, des arylphénols, ou parmi des alkylglucosides, des polysorbates, des cocamides.

Selon un mode de réalisation de l'invention, l'agent tensioactif est le laurylsulfate de sodium (SLS ou SDS).

De préférence, la phase continue aqueuse d'une dispersion selon l'invention peut comprendre une teneur massique en tensioactif(s) supérieure à 0,001%, et avantageusement supérieure à 0,1%, en poids par rapport au poids total de ladite dispersion.

Par ailleurs, la phase continue aqueuse d'une dispersion selon l'invention peut comprendre une teneur massique en tensioactif(s) inférieure à 10,0%, et avantageusement inférieure à 1,0%, en poids par rapport au poids total de ladite dispersion.

### *Ecorce des gouttes*

Comme mentionné précédemment, les gouttes selon l'invention sont entourées d'une écorce (ou membrane) comprenant au moins un polymère anionique et au moins un polymère cationique.

Selon l'invention, les gouttes obtenues peuvent présenter une écorce très fine, notamment d'épaisseur inférieure à 1% du diamètre des gouttes.

L'épaisseur de l'écorce est ainsi de préférence inférieure à 1 $\mu$m et est donc trop faible pour être mesurée par des méthodes optiques.

Selon un mode de réalisation, l'épaisseur de l'écorce des gouttes est inférieure à 1 000 nm, notamment comprise de 1 à 500 nm, de préférence inférieure à 100 nm, avantageusement inférieure à 50 nm, préférentiellement inférieure à 10 nm.

La mesure de l'épaisseur de l'écorce des gouttes de l'invention peut être effectuée par la méthode de diffusion de neutrons aux petits angles (Small-Angle X-ray Scattering), telle que mise en œuvre dans Sato et al. J. Chem. Phys. 111, 1393-1401 (2007).

Pour cela, les gouttes sont produites en utilisant de l'eau deutérée, puis sont lavées trois fois avec une huile deutérée, comme par exemple une huile deutérée de type hydrocarboné (octane, dodécane, hexadécane).

Après lavage, les gouttes sont ensuite transférées dans la cellule de Neutrons afin de déterminer le spectre I(q) ; q étant le vecteur d'onde.

A partir de ce spectre, on applique les traitements analytiques classiques (REF) afin de déterminer l'épaisseur de l'écorce hydrogénée (non deutérée).

Selon un mode de réalisation, l'écorce entourant les gouttes de la phase dispersée est rigidifiée, ce qui confère notamment une bonne résistance aux gouttes et diminue, voire empêche, leur coalescence.

Cette écorce est typiquement formée par coacervation, c'est-à-dire par précipitation de polymères chargés de charges opposées. Au sein d'un coacervat, les liaisons liant les polymères chargés entre eux sont de type ionique, et sont généralement plus fortes que des liaisons présentes au sein d'une membrane de type tensioactif.

L'écorce est formée par coacervation d'au moins deux polymères chargés de polarité opposée (ou polyélectrolyte) et de préférence en présence d'un premier polymère, de type cationique, et d'un deuxième polymère, différent du premier polymère, de type anionique. Ces deux polymères jouent le rôle d'agents de rigidification de la membrane.

La formation du coacervat entre ces deux polymères est généralement provoquée par une modification des conditions du milieu réactionnel (température, pH, concentration en réactifs, etc.). La réaction de coacervation résulte de la neutralisation de ces deux polymères chargés de polarités opposées et permet la formation d'une structure membranaire par interactions électrostatiques entre le polymère anionique et le polymère cationique. La membrane ainsi formée autour de chaque goutte forme typiquement une écorce qui encapsule totalement le cœur de la goutte comprenant l'(les) agent(s) gélifiant(s), et isole ainsi le cœur de la goutte de la phase aqueuse continue.

*Polymère anionique*

Dans le cadre de la présente description, on entend par "polymère de type anionique" ou « polymère anionique » un polymère comportant des fonctions chimiques de type anionique. On peut aussi parler de polyélectrolyte anionique.

Par "fonction chimique de type anionique", on entend une fonction chimique AH capable de céder un proton pour donner une fonction $A^-$. Selon les conditions du milieu dans lequel il se trouve, le polymère de type anionique comporte donc des fonctions chimiques sous forme AH, ou bien sous forme de sa base conjuguée $A^-$.

Comme exemple de fonctions chimiques de type anionique, on peut citer les fonctions acide carboxylique -COOH, éventuellement présentes sous forme d'anion carboxylate $-COO^-$.

Comme exemple de polymère de type anionique, on peut citer tout polymère formé par la polymérisation de monomères dont au moins une partie porte des fonctions chimiques de type anionique, tel que des fonctions acide carboxylique. De tels monomères sont par exemple l'acide acrylique, l'acide maléique, ou tout monomère éthyléniquement insaturé comportant au moins une fonction acide carboxylique. Il peut par exemple s'agir de polymère anionique comprenant des unités monomères comportant au moins une fonction chimique de type acide carboxylique.

De préférence, le polymère anionique est hydrophile, c'est-à-dire soluble ou dispersible dans l'eau.

Parmi les exemples de polymère de type anionique appropriés à la mise en œuvre de l'invention, on peut citer les copolymères d'acide acrylique ou d'acide maléique et d'autres monomères, tels que l'acrylamide, les acrylates d'alkyle, les acrylates d'alkyle en $C_5$-$C_8$, les acrylates d'alkyle en $C_{10}$-$C_{30}$, les méthacrylates d'alkyle en $C_{12}$-$C_{22}$, les méthacrylates méthoxypolyéthylèneglycol, les acrylates d'hydroxyester, les acrylates crosspolymères, et leurs mélanges.

Selon l'invention, un polymère de type anionique est de préférence un carbomère tel que décrit ci-après. Ce polymère peut également être un copolymère réticulé acrylates/$C_{10}$-$_{30}$ alkyl acrylate (nom INCI : acrylates/$C_{10}$-$_{30}$ alkyl acrylate Crosspolymer).

Selon un mode de réalisation, l'écorce des gouttes comprend au moins un polymère anionique, tel que par exemple un carbomère.

Dans le cadre de l'invention, et sauf mention contraire, on entend par "carbomère", un homopolymère éventuellement réticulé, issu de la polymérisation de l'acide acrylique. Il s'agit donc d'un poly(acide acrylique) éventuellement réticulé. Parmi les carbomères de l'invention, on peut citer ceux commercialisés sous les noms Tego®Carbomer 340FD de Evonik, Carbopol® 981 de Lubrizol, Carbopol ETD 2050 de Lubrizol, ou encore Carbopol Ultrez 10 de Lubrizol.

Selon un mode de réalisation, on entend par "carbomère" ou "carbomer" ou "Carbopol®", un polymère d'acide acrylique de haut poids moléculaire réticulé avec du sucrose allylique ou des éthers allyliques de pentaérythritol (Handbook of Pharmaceutical Excipients, 5$^{eme}$ Edition, plll). Par exemple, il s'agit du Carbopol®910, du Carbopol®934, Carbopol®934P, du Carbopol®940, du Carbopol®941, du Carbopol®71 G, du Carbopol®980, du Carbopol®971P ou du Carbopol®974P. Selon un mode de réalisation, la viscosité dudit carbomère est comprise entre 4 000 et 60 000 cP à 0,5% w/w.

Les carbomères ont d'autres dénominations : acides polyacryliques, polymères carboxyvinyliques ou carboxy polyéthylènes.

Une dispersion selon l'invention peut comprendre de 0,01% à 5% en poids, de préférence de 0,05% à 2%, et préférentiellement de 0,10% à 0,5%, de polymère(s) anionique(s), notamment de carbomère(s), par rapport au poids total de ladite dispersion.

Selon l'invention, les dispersions selon l'invention peuvent comprendre un carbomère et un copolymère réticulé acrylates/$C_{10}$-$_{30}$ alkyl acrylate.

La phase aqueuse selon l'invention peut également comprendre au moins un polymère réticulé ou au moins

un copolymère réticulé, ledit polymère réticulé ou copolymère réticulé comprenant au moins une unité dérivée de la polymérisation d'un des monomères suivants : acide acrylique ou méthacrylique, acrylate ou méthacrylate d'alkyle comprenant de 1 à 30 atomes de carbone, ou leurs sels.

La phase aqueuse peut également comprendre un mélange de polymères réticulés ou un mélange de copolymères réticulés ou encore un mélange de polymère(s) réticulé(s) et de copolymère(s) réticulé(s).

Selon l'invention, le terme "unité dérivée de la polymérisation d'un monomère" signifie que le polymère ou copolymère est un polymère ou copolymère obtenu par polymérisation ou copolymère dudit monomère.

Selon un mode de réalisation, le polymère réticulé ou le copolymère réticulé est un polyacrylate réticulé.

Les copolymères et polymères réticulés de l'invention sont anioniques.

Selon un mode de réalisation, le copolymère est un copolymère d'acide carboxylique insaturé et de carboxylate insaturé d'alkyle en $C_{1-30}$, de préférence en $C_1$-$C_4$. Un tel copolymère comporte au moins un motif hydrophile de type acide carboxylique insaturé oléfinique et au moins un motif hydrophobe de type ester d'alkyle ($C_1$-$C_{30}$) d'acide carboxylique insaturé.

De préférence, ces copolymères sont choisis parmi ceux dont le motif hydrophile de type acide carboxylique insaturé oléfinique correspond au monomère de formule (I) suivante :

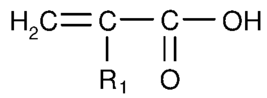

(I)

dans laquelle : $R_1$ désigne H ou $CH_3$ ou $C_2H_5$, c'est-à-dire des motifs acide acrylique, acide méthacrylique ou acide éthacrylique,

et dont le motif hydrophobe de type ester d'alkyle ($C_1$-$C_{30}$) d'acide carboxylique insaturé correspond au monomère de formule (II) suivante :

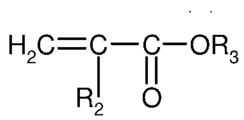

(II)

dans laquelle : $R_2$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R_3$ désignant un radical alkyle en $C_1$-$C_{30}$, et de préférence en $C_1$-$C_4$.

Parmi ce type de copolymères, on utilisera plus particulièrement ceux formés à partir d'un mélange de monomères comprenant :

(i) essentiellement de l'acide acrylique,

(ii) un ester de formule (II) décrite ci-dessus et dans laquelle $R_2$ désigne H ou $CH_3$, $R_3$ désignant un radical alkyle ayant de 1 à 4 atomes de carbone,

(iii) et un agent réticulant, qui est un monomère insaturé polyéthylénique copolymérisable bien connu, comme le phtalate de diallyle, le triméthylolpropane tri(meth)acrylate, diallyl itaconate, diallyl fumarate, diallyl maléate, zinc (meth)acrylate, le (méth)acrylate d'allyle, le divinylbenzène, le diméthacrylate de (poly)éthylèneglycol, le méthylène-bis-acrylamide, et l'huile de ricin.

Selon un mode de réalisation, le polymère réticulé ou le copolymère réticulé est un polymère ou copolymère d'acide acrylique et/ou d'acide méthacrylique, et/ou d'acrylate d'alkyle comprenant de 1 à 30 atomes de carbone, de préférence de 1 à 4 atomes de carbone, et/ou de méthacrylate d'alkyle comprenant de 1 à 30 atomes de carbone, de préférence de 1 à 4 atomes de carbone.

Selon un mode de réalisation, le copolymère réticulé est un copolymère réticulé d'acide méthacrylique et d'acrylate d'alkyle comprenant de 1 à 4 atomes de carbone, de préférence 2 atomes de carbone.

Dans le cadre de l'invention, et sauf mention contraire, on entend par « copolymère réticulé d'acide méthacrylique et d'acrylate d'alkyle comprenant de 1 à 4 atomes de carbones », un copolymère réticulé résultant de la polymérisation d'un monomère d'acide méthacrylique et d'un monomère d'acrylate d'alkyle comprenant de 1 à 4 atomes de carbones.

De préférence, dans ce copolymère, l'acide méthacrylique représente de 20% à 80% en poids, de préférence de 35% à 65% en poids du poids total du copolymère.

De préférence, dans ce copolymère, l'acrylate d'alkyle représente de 15% à 80% en poids, de préférence de

35% à 65% en poids du poids total du copolymère.

En particulier, l'acrylate d'alkyle est choisi parmi le méthacrylate d'alkyle, l'acrylate d'éthyle et l'acrylate de butyle.

Selon un mode de réalisation, le polymère réticulé ou le copolymère réticulé selon l'invention, présent dans la phase aqueuse continue, est choisi dans le groupe constitué des polymères ou copolymères suivants : Acrylates Copolymer, Acrylates crosspolymer-4, Acrylates crosspolymer-3, Polyacrylate-2 Crosspolymer et Polyacrylate-14 (noms INCI).

Parmi lesdits polymères ci-dessus, on préfère tout particulièrement, selon la présente invention, les produits vendus par la société LUBRIZOL sous les dénominations commerciales Fixate Superhold (nom INCI = Polyacrylate-2 Crosspolymer), Fixate Freestyle Polymer (nom INCI = Acrylates crosspolymer-3), Carbopol® Aqua SF1 (nom INCI = Acrylates copolymer) et Carbopol® Aqua SF2 (nom INCI = Acrylates crosspolymer-4).

De préférence, le copolymère réticulé est le Carbopol® Aqua SF1 (nom INCI = Acrylates copolymer).

Selon un mode de réalisation, le copolymère réticulé est choisi parmi les copolymères réticulés d'acide acrylique ou méthacrylique et d'acrylates d'alkyle comprenant de 1 à 4 atomes de carbone.

Selon l'invention, la dispersion de l'invention peut comprendre de 0,1% à 10% en poids, de préférence de 0,5% à 8% en poids, et préférentiellement de 1% à 3% en poids de polymère(s) réticulé(s) ou copolymère(s) réticulé(s) par rapport au poids total de ladite dispersion.

Selon l'invention, les dispersions selon l'invention peuvent comprendre un carbomère et un copolymère réticulé Carbopol® Aqua SF1 (nom INCI = Acrylates copolymer).

*Polymère cationique*

Selon un mode de réalisation, les gouttes, et notamment l'écorce desdites gouttes, comprennent en outre un polymère de type cationique. Elles peuvent également comprendre plusieurs polymères de type cationique. Ce polymère cationique est celui mentionné ci-dessus qui forme l'écorce par coacervation avec le polymère anionique.

Dans le cadre de la présente demande, et sauf mention contraire, on entend par "polymère de type cationique" ou « polymère cationique » un polymère comportant des fonctions chimiques de type cationique. On peut aussi parler de polyélectrolyte cationique.

De préférence, le polymère cationique est lipophile ou liposoluble.

Dans le cadre de la présente demande, et sauf mention contraire, par "fonction chimique de type cationique", on entend une fonction chimique B capable de capter un proton pour donner une fonction $BH^+$. Selon les conditions du milieu dans lequel il se trouve, le polymère de type cationique comporte donc des fonctions chimiques sous forme B, ou bien sous forme $BH^+$, son acide conjugué.

Comme exemple de fonctions chimiques de type cationique, on peut citer les fonctions amine primaire, secondaire et tertiaire, éventuellement présentes sous forme de cations ammoniums.

Comme exemple de polymère de type cationique, on peut citer tout polymère formé par la polymérisation de monomères dont au moins une partie porte des fonctions chimiques de type cationique, tel que des fonctions amine primaire, secondaire ou tertiaire.

De tels monomères sont par exemple l'aziridine, ou tout monomère éthyléniquement insaturé comportant au moins une fonction amine primaire, secondaire ou tertiaire.

Parmi les exemples de polymères cationiques appropriés à la mise en œuvre de l'invention, on peut citer l'amodiméthicone, dérivé d'un polymère silicone (polydiméthylsiloxane, aussi appelé diméthicone), modifié par des fonctions amine primaire et amine secondaire.

On peut également citer des dérivés de l'amodiméthicone, comme par exemple des copolymères de l'amodiméthicone, l'aminopropyl diméthicone, et plus généralement des polymères silicones linéaires ou ramifiés comportant des fonctions amines.

On peut citer le copolymère de bis-isobutyl PEG-14/amodiméthicone, le Bis (C13-15 Alkoxy) PG-Amodimethicone, le Bis-Cetearyl Amodimethicone et le bis-hydroxy/méthoxy amodiméthicone.

On peut également citer les polymères de type polysaccharide comprenant des fonctions amine, tel que le chitosan ou les dérivés de gomme guar (chlorure d'hydroxypropyltrimonium guar).

On peut également citer les polymères de type polypeptide comprenant des fonctions amine, tel que la polylysine.

On peut également citer les polymères de type polyéthylèneimine comprenant des fonctions amine, tel que la polyéthylèneimine linéaire ou branchée.

Selon un mode de réalisation, les gouttes, et notamment l'écorce desdites gouttes, comprennent un polymère cationique qui est un polymère silicone modifié par une fonction amine primaire, secondaire ou tertiaire, tel que l'amodiméthicone.

Selon un mode de réalisation, les gouttes, et en particulier l'écorce desdites gouttes, comprennent de l'amodiméthicone.

Selon un mode de réalisation particulièrement préféré, le polymère cationique répond à la formule suivante :

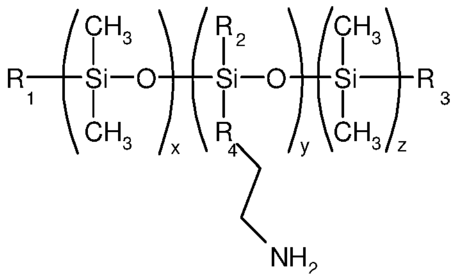

dans laquelle :

- $R_1$, $R_2$ et $R_3$, indépendamment les uns des autres, représentent OH ou $CH_3$ ;
- $R_4$ représente un groupe $-CH_2-$ ou un groupe -X-NH- dans lequel X est un radical alkylène divalent en C3 ou C4 ;
- x est un nombre entier compris entre 10 et 5 000, de préférence entre 30 et 1 000, et mieux entre 80 et 300 ;
- y est un nombre entier compris entre 2 et 1 000, de préférence entre 4 et 100, et mieux entre 5 et 20 ; et
- z est un nombre entier compris entre 0 et 10, de préférence entre 0 et 1, et mieux est égal à 1.

Dans la formule susmentionnée, lorsque $R_4$ représente un groupe -X-NH-, X est relié à l'atome de silicium.

Dans la formule susmentionnée, $R_1$, $R_2$ et $R_3$ représentent de préférence $CH_3$. Dans la formule susmentionnée, $R_4$ est de préférence un groupe $-(CH_2)_3-NH-$.

Selon l'invention, chaque goutte peut comprendre de 0,01% à 10%, de préférence de 0,05% à 5%, en poids de polymère(s) cationique(s), notamment d'amodiméthicone(s), par rapport au poids total de la phase grasse.

***Phase grasse***

Selon l'invention, les dispersions comprennent une phase grasse dispersée, sous forme de gouttes, comprenant au moins un agent gélifiant.

*Agent gélifiant*

Comme indiqué précédemment, la présente invention repose sur la présence, au niveau de la phase grasse dispersée, d'au moins un agent gélifiant. Un tel agent gélifiant est différent des polymères anioniques et cationiques décrits ci-dessus.

Dans le cadre de l'invention, et sauf mention contraire, on entend par « agent gélifiant », un agent permettant d'augmenter la viscosité de la phase grasse des gouttes de la dispersion dépourvue dudit agent gélifiant, et d'atteindre une viscosité finale de la phase grasse gélifiée supérieure à 20 000 mPa.s, de préférence supérieure à 50 000 mPa.s, mieux supérieure à 100 000 mPa.s, et tout particulièrement supérieure à 200 000 mPa.s.

De préférence, la viscosité de la phase grasse des gouttes de la dispersion en présence dudit agent gélifiant est comprise entre 20 000 et 100 000 000 mPa.s, de préférence entre 50 000 et 1 000 000 mPa.s, et mieux entre 100 000 à 500 000 mPa.s, à 25°C.

Le choix en agent(s) gélifiant(s) s'effectue notamment au regard de la nature de la phase dispersée. Ainsi, pour des raisons évidentes de compatibilités, l'agent gélifiant est lipophile.

Selon un mode de réalisation, l'agent gélifiant est choisi parmi les agents gélifiants lipophiles tels que décrits ci-après, les corps gras solides à température et pression ambiante, notamment choisis parmi les cires, les corps gras pâteux, les beurres, et leurs mélanges.

*Agent(s) gélifiant(s) lipophile(s)*

Les gélifiants utilisables selon l'invention peuvent être des gélifiants lipophiles organiques ou minéraux, polymériques ou moléculaires.

Comme gélifiant lipophile minéral, on peut citer les argiles éventuellement modifiées comme les hectorites modifiées par un chlorure d'ammonium en $C_{10}$ à $C_{22}$, comme l'hectorite modifiée par du chlorure de di-stéaryl di-méthyl ammonium telle que, par exemple, celle commercialisée sous la dénomination de Bentone 38V® par la société ELEMENTIS. On peut également citer l'hectorite modifiée par du chlorure de distéaryldiméthylammonium, connue également comme bentonite de quaternium-18, telle que les produits commercialisés ou fabriqués sous les dénominations Bentone

34 par la société Rheox, Claytone XL, Claytone 34 et Claytone 40 commercialisés ou fabriqués par la société Southern Clay, les argiles modifiées connues sous la dénomination de bentonites de benzalkonium et de quaternium-18 et commercialisées ou fabriquées sous les dénominations Claytone HT, Claytone GR et Claytone PS par la société Southern Clay, les argiles modifiées par du chlorure de stéaryldiméthylbenzoylammonium, connues comme bentonites de stéralkonium, telles que les produits commercialisés ou fabriqués sous les dénominations Claytone APA et Claytone AF par la société Southern Clay, et Baragel 24 commercialisé ou fabriqué par la société Rheox.

On peut également citer la silice pyrogénée éventuellement traitée hydrophobe en surface dont la taille des particules est inférieure à 1 μm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe.

Les groupements hydrophobes peuvent être :

- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (8$^{ème}$ édition, 2000). Elles sont par exemple commercialisées sous les références Aerosil R812® par la société DEGUSSA, CAB-O-SIL TS-530® par la société CABOT ; ou
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (8$^{ème}$ édition, 2000). Elles sont par exemple commercialisées sous les références Aerosil R972®, et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Dow Corning® EL-7040, de Trefil E-505C® et de Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Selon un mode de réalisation, les gélifiants utilisables selon l'invention peuvent être choisi dans le groupe constitué des polyacrylates, des esters de dextrine et d'acide(s) gras, des esters de glycérol et d'acide(s) gras, des polyamides, et de leurs mélanges.

Comme gélifiant lipophile, on peut encore citer les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO 02/056847, WO 02/47619, en particulier les résines de polyamides (notamment comprenant des groupes alkyles ayant de 12 à 22 atomes de carbone) telles que celles décrites dans US 5783657.

A titre d'exemple de résine de polyamide pouvant être mise en œuvre selon la présente invention, on peut citer UNICLEAR 100 VG® commercialisé par la société ARIZONA CHEMICAL.

On peut également utiliser les polyamides siliconés du type polyorganosiloxane tels que ceux décrits dans US 5 874 069, US 5 919 441, US 6 051 216 et US 5 981 680.

Ces polymères siliconés peuvent appartenir aux deux familles suivantes :

- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

Parmi les gélifiants lipophiles pouvant être utilisés dans la présente invention, on peut encore citer les esters de dextrine et d'acide gras, tels que les palmitates de dextrine.

Selon un mode de réalisation, l'ester de dextrine et d'acide(s) gras selon l'invention est un mono- ou poly-ester de dextrine et d'au moins un acide gras répondant à la formule (II) suivante :

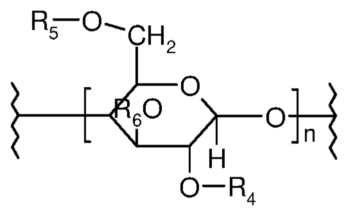

(II)

dans laquelle :

- n est un nombre entier allant de 2 à 200, de préférence allant de 20 à 150, et en particulier allant de 25 à 50,
- les radicaux $R_4$, $R_5$ et $R_6$, identiques ou différents, sont choisis parmi l'hydrogène ou un groupement acyle -$COR_a$ dans lequel le radical $R_a$ représente un radical hydrocarboné, linéaire ou ramifié, saturé ou insaturé, possédant de 5 à 50, de préférence de 5 à 25 atomes de carbone,
sous réserve qu'au moins un desdits radicaux $R_4$, $R_5$ ou $R_6$ est différent de l'hydrogène.

Selon un mode de réalisation, $R_4$, $R_5$ et $R_6$ représentent, indépendamment les uns des autres, H ou un groupement acyle -$COR_a$ dans lequel $R_a$ est un radical hydrocarboné tel que défini précédemment, sous réserve qu'au moins deux desdits radicaux $R_4$, $R_5$ ou $R_6$ sont identiques et différents de l'hydrogène.

Selon un mode de réalisation, lorsque les radicaux $R_4$, $R_5$ et $R_6$, identiques ou différents, représentent un radical -$COR_a$, ceux-ci peuvent être choisis parmi les radicaux caprylyle, caproyle, lauroyle, myristyle, palmityle, stéaryle, eicosanyle, docosanoyle, isovaléryle, éthyl-2 butyryle, éthylméthylacétyle, isoheptanyle, éthyl-2 hexanyle, isononanyle, isodécanyle, isotridécanyle, isomyristyle, isopalmityle, isostéaryle, isohexanyle, décènyle, dodécenyle, tétradécényle, myristyle, hexadécénoyle, palmitoléyle, oléyle, élaidyle, eicosényle, sorbyle, linoléyle, linolényle, punicyle, arachidonyle, stéarolyle, et leurs mélanges.

Parmi les esters de dextrine et d'acide(s) gras, on peut par exemple citer les palmitates de dextrine, les myristates de dextrine, les palmitates/éthylhexanoates de dextrine et leurs mélanges.

On peut notamment citer les esters de dextrine et d'acide(s) gras commercialisés sous les dénominations Rheopearl® KL2 (nom INCI : dextrin palmitate), Rheopearl® TT2 (nom INCI : dextrin palmitate ethylhexanoate), et Rheopearl® MKL2 (nom INCI : dextrin myristate) par la société Miyoshi Europe.

Selon un mode de réalisation, l'agent gélifiant est choisi parmi les polyacrylates résultant de la polymérisation d'acrylate(s) d'alkyle en $C_{10}$-$C_{30}$, de préférence d'acrylate(s) d'alkyle en $C_{14}$-$C_{24}$, et encore plus préférentiellement d'acrylate(s) d'alkyle en $C_{18}$-$C_{22}$.

Selon un mode de réalisation, les polyacrylates sont des polymères d'acide acrylique estérifié avec un alcool gras dont la chaîne carbonée saturée comprend de 10 à 30 atomes de carbone, de préférence de 14 à 24 atomes de carbone, ou un mélange desdits alcools gras. De préférence, l'alcool gras comprend 18 atomes de carbone ou 22 atomes de carbone.

Parmi les polyacrylates, on peut citer plus particulièrement le polyacrylate de stéaryle, le polyacrylate de béhényle. De préférence, l'agent gélifiant est le polyacrylate de stéaryle ou le polyacrylate de béhényle.

On peut notamment citer les polyacrylates commercialisés sous les dénominations Interlimer® (nom INCI : Poly $C_{10}$-$C_{30}$ alkyl acrylate), notamment Interlimer® 13.1 et Interlimer® 13.6, par la société Airproducts.

Selon un mode de réalisation, l'agent gélifiant est un ester de glycérol et d'acide(s) gras, en particulier un mono-, di- ou triester de glycérol et d'acide(s) gras. Typiquement, ledit ester de glycérol et d'acide(s) gras peut être utilisé seul ou en mélange.

Selon l'invention, il peut s'agir d'un ester de glycérol et d'un acide gras ou d'un ester de glycérol et d'un mélange d'acides gras.

Selon un mode de réalisation, l'acide gras est choisi dans le groupe constitué de l'acide béhénique, de l'acide isooctadécanoique, de l'acide stéarique, de l'acide eicosanoïque, et de leurs mélanges.

Selon un mode de réalisation, l'ester de glycérol et d'acide(s) gras présente la formule (I) suivante :

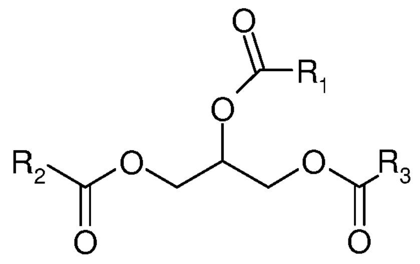

(I)

dans laquelle : $R_1$, $R_2$ et $R_3$ sont, indépendamment l'un de l'autre, choisi parmi H et une chaîne alkyle saturée comprenant de 4 à 30 atomes de carbone, au moins un de $R_1$, $R_2$ et $R_3$ étant différent de H.

Selon un mode de réalisation, $R_1$, $R_2$ et $R_3$ sont différents.

Selon un mode de réalisation, $R_1$, $R_2$ et/ou $R_3$ représente une chaîne alkyle saturée comprenant de 4 à 30, de préférence de 12 à 22, et préférentiellement de 18 à 22 atomes de carbone.

Selon un mode de réalisation, l'ester de glycérol et d'acide(s) gras correspond à un composé de formule (I) dans laquelle $R_1$ = H, $R_2$ = $C_{21}H_{43}$ et $R_3$ = $C_{19}H_{40}$.

Selon un mode de réalisation, l'ester de glycérol et d'acide(s) gras correspond à un composé de formule (I) dans laquelle $R_1$ = $R_2$ = $R_3$ = $C_{21}H_{43}$.

Selon un mode de réalisation, l'ester de glycérol et d'acide(s) gras correspond à un composé de formule (I) dans laquelle $R_1$ = $R_2$ = H, et $R_3$ = $C_{19}H_{40}$.

Selon un mode de réalisation, l'ester de glycérol et d'acide(s) gras correspond à un composé de formule (I) dans laquelle $R_1$ = $R_2$ = H, et $R_3$ = $C_{17}H_{35}$.

On peut notamment citer les esters de glycérol et d'acide(s) gras commercialisés sous les dénominations Nomcort HK-G (nom INCI : Glyceryl behenate/eicosadioate) et Nomcort SG (nom INCI : Glyceryl tribehenate, isostearate, eicosadioate), par la société Nisshin Oillio.

*Cire(s)*

Par « cire », on entend au sens de l'invention, un composé lipophile, solide à température ambiante (25°C), à changement d'état solide/liquide réversible, ayant un point de fusion supérieur ou égal à 30 °C pouvant aller jusqu'à 120°C.

Le protocole de mesure de ce point de fusion est décrit plus loin.

Les cires susceptibles d'être utilisées dans une composition selon l'invention peuvent être choisies parmi les cires, solides, déformables ou non à température ambiante, d'origine animale, végétale, minérale ou de synthèse et leurs mélanges.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut notamment citer les cires commercialisées sous les dénominations Kahlwax®2039 (nom INCI : Candelilla cera) et Kahlwax®6607 (nom INCI : Helianthus Annuus Seed Wax) par la société Kahl Wachsraffinerie, Casid HSA (nom INCI : Hydroxystearic Acid) par la société SACI CFPA, Performa®260 (nom INCI : Synthetic wax) et Performa®103 (nom INCI : Synthetic wax) par la société New Phase, et AJK-CE2046 (nom INCI : Cetearyl alcohol, dibutyl lauroyl glutamide, dibutyl ethylhaxanoyl glutamide) par la société Kokyu Alcohol Kogyo.

On peut aussi citer les cires obtenues par hydrogénation catalytique d'huiles animales ou végétales ayant des chaînes grasses, linéaires ou ramifiées, en $C_8$-$C_{32}$.

Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.

On peut également utiliser les cires obtenues par transestérification et hydrogénation d'huiles végétales, telles que l'huile de ricin ou d'olive, comme les cires vendues sous les dénominations de Phytowax ricin 16L64® et 22L73® et Phytowax Olive 18L57 par la société SOPHIM. De telles cires sont décrites dans la demande FR-A-2792190.

On peut aussi utiliser des cires siliconées qui peuvent être avantageusement des polysiloxanes substitués, de préférence à bas point de fusion.

Parmi les cires de silicones commerciales de ce type, on peut citer notamment celles vendues sous les dénominations Abilwax 9800, 9801 ou 9810 (GOLDSCHMIDT), KF910 et KF7002 (SHIN ETSU), ou 176-1118-3 et 176-11481

(GENERAL ELECTRIC).

Les cires de silicone utilisables peuvent également être des alkyl ou alcoxydiméthicones tels que les produits commerciaux suivants : Abilwax 2428, 2434 et 2440 (GOLDSCHMIDT), ou VP 1622 et VP 1621 (WACKER), ainsi que les ($C_{20}$-$C_{60}$) alkyldiméthicones, en particulier les ($C_{30}$-$C_{45}$) alkyldiméthicones comme la cire siliconée vendue sous la dénomination SF-1642 par la société GE-Bayer Silicones.

On peut également utiliser des cires hydrocarbonées modifiées par des groupements siliconés ou fluorés comme par exemple : siliconyl candelilla, siliconyl beeswax et Fluorobeeswax de Koster Keunen.

Les cires peuvent également être choisies parmi les cires fluorées.

*Beurre(s) ou corps gras pâteux*

Par « beurre » (également appelé « corps gras pâteux ») au sens de la présente invention, on entend un composé gras lipophile à changement d'état solide/liquide réversible et comportant à la température de 25°C une fraction liquide et une fraction solide, et à pression atmosphérique (760 mm Hg). En d'autres termes, la température de fusion commençante du composé pâteux peut être inférieure à 25°C. La fraction liquide du composé pâteux mesurée à 25°C peut représenter de 9% à 97 % en poids du composé. Cette fraction liquide à 25°C représente de préférence entre 15% et 85 %, de préférence encore entre 40 et 85 % en poids. De préférence, le ou les beurres présentent une température de fin de fusion inférieure à 60°C. De préférence, le ou les beurres présentent une dureté inférieure ou égale à 6 MPa.

De préférence, les beurres ou corps gras pâteux présentent à l'état solide une organisation cristalline anisotrope, visible par observations aux rayons X.

Au sens de l'invention, la température de fusion correspond à la température du pic le plus endothermique observé en analyse thermique (DSC) telle que décrite dans la norme ISO 11357-3 ; 1999. Le point de fusion d'un pâteux ou d'une cire peut être mesuré à l'aide d'un calorimètre à balayage différentiel (DSC), par exemple le calorimètre vendu sous la dénomination "DSC Q2000" par la société TA Instruments.

Concernant la mesure de la température de fusion et la détermination de la température de fin de fusion, les protocoles de préparation des échantillons et de mesure sont les suivants : Un échantillon de 5 mg de corps gras pâteux (ou beurre) ou de cire préalablement chauffé à 80°C et prélevés sous agitation magnétique à l'aide d'une spatule également chauffée est placé dans une capsule hermétique en aluminium, ou creuset. Deux essais sont réalisés pour s'assurer de la reproductibilité des résultats.

Les mesures sont réalisées sur le calorimètre mentionné ci-dessus. Le four est soumis à un balayage d'azote. Le refroidissement est assuré par l'échangeur thermique RCS 90. L'échantillon est ensuite soumis au protocole suivant en étant tout d'abord mis en température à 20°C, puis soumis à une première montée en température allant de 20°C à 80°C, à la vitesse de chauffe de 5°C/minute, puis est refroidi de 80°C à -80°C à une vitesse de refroidissement de 5°C/minute et enfin soumis à une deuxième montée en température allant de -80°C à 80°C à une vitesse de chauffe de 5°C/minute. Pendant la deuxième montée en température, on mesure la variation de la différence de puissance absorbée par le creuset vide et par le creuset contenant l'échantillon de beurre en fonction de la température. Le point de fusion du composé est la valeur de la température correspondant au sommet du pic de la courbe représentant la variation de la différence de puissance absorbée en fonction de la température. La température de fin de fusion correspond à la température à laquelle 95% de l'échantillon a fondu.

La fraction liquide en poids du beurre (ou corps gras pâteux) à 25°C est égale au rapport de l'enthalpie de fusion consommée à 25°C sur l'enthalpie de fusion du beurre. L'enthalpie de fusion du beurre ou composé pâteux est l'enthalpie consommée par le composé pour passer de l'état solide à l'état liquide.

Le beurre est dit à l'état solide lorsque l'intégralité de sa masse est sous forme solide cristalline. Le beurre est dit à l'état liquide lorsque l'intégralité de sa masse est sous forme liquide. L'enthalpie de fusion du beurre est égale à l'intégrale de l'ensemble de la courbe de fusion obtenue à l'aide du calorimètre suscité, avec une montée en température de 5°C ou 10°C par minute, selon la norme ISO 11357-3:1999. L'enthalpie de fusion du beurre est la quantité d'énergie nécessaire pour faire passer le composé de l'état solide à l'état liquide. Elle est exprimée en J/g.

L'enthalpie de fusion consommée à 25°C est la quantité d'énergie absorbée par l'échantillon pour passer de l'état solide à l'état qu'il présente à 25°C constitué d'une fraction liquide et d'une fraction solide. La fraction liquide du beurre mesurée à 32°C représente de préférence de 30% à 100 % en poids du composé, de préférence de 50% à 100%, de préférence encore de 60% à 100 % en poids du composé. Lorsque la fraction liquide du beurre mesurée à 32°C est égale à 100%, la température de la fin de la plage de fusion du composé pâteux est inférieure ou égale à 32°C. La fraction liquide du beurre mesurée à 32°C est égale au rapport de l'enthalpie de fusion consommée à 32°C sur l'enthalpie de fusion du beurre. L'enthalpie de fusion consommée à 32°C est calculée de la même façon que l'enthalpie de fusion consommée à 23°C.

Concernant la mesure de la dureté, les protocoles de préparation des échantillons et de mesure sont les suivants : la composition selon l'invention ou le beurre est placé dans un moule de 75 mm de diamètre qui est rempli à environ 75% de sa hauteur. Afin de s'affranchir du passé thermique et de contrôler la cristallisation, le moule est placé

à l'étuve programmable Vôtsch VC0018 où il est tout d'abord mis en température à 80°C pendant 60 minutes, puis refroidi de 80°C à 0°C à une vitesse de refroidissement de 5°C/minute, puis laissé à la température stabilisée de 0°C pendant 60 minutes, puis soumis à une montée en température allant de 0°C à 20°C, à une vitesse de chauffe de 5°C/minute, puis laissé à la température stabilisée de 20°C pendant 180 minutes. La mesure de la force de compression est réalisée avec le texturomètre TA/TX2i de Swantech. Le mobile utilisé est choisi selon la texture : - mobile cylindrique en acier de 2 mm de diamètre pour les matières premières très rigides ; - mobile cylindrique en acier de 12 mm de diamètre pour les matières premières peu rigides. La mesure comporte 3 étapes : une 1ère étape après détection automatique de la surface de l'échantillon où le mobile se déplace à la vitesse de mesure de 0,1 mm/s, et pénètre dans la composition selon l'invention ou le beurre à une profondeur de pénétration de 0,3 mm, le logiciel note la valeur de la force maximale atteinte ; une 2ème étape dite de relaxation ou le mobile reste à cette position pendant une seconde et où on note la force après 1 seconde de relaxation ; enfin une 3ème étape dite de retrait ou le mobile revient à sa position initiale à la vitesse de 1 mm/s et on note l'énergie de retrait de la sonde (force négative).

La valeur de la dureté mesurée lors de la première étape correspond à la force de compression maximale mesurée en Newton divisée par la surface du cylindre du texturomètre exprimée en $mm^2$ en contact avec le beurre ou la composition selon l'invention. La valeur de dureté obtenue est exprimée en méga-pascals ou MPa.

Le corps gras pâteux ou beurre peut être choisi parmi les composés synthétiques et les composés d'origine végétale. Un corps gras pâteux peut être obtenu par synthèse à partir de produits de départ d'origine végétale.

Le corps gras pâteux est avantageusement choisi parmi :

- la lanoline et ses dérivés tels que l'alcool de lanoline, les lanolines oxyéthylénées, la lanoline acétylée, les esters de lanoline tels que le lanolate d'isopropyle, les lanolines oxypropylénées,
- les composés siliconés polymères ou non-polymères comme les polydiméthysiloxanes de masses moléculaires élevées, les polydiméthysiloxanes à chaînes latérales du type alkyle ou alcoxy ayant de 8 à 24 atomes de carbone, notamment les stéaryl diméthicones,
- les composés fluorés polymères ou non-polymères,
- les polymères vinyliques, notamment
- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyle ayant de préférence un groupement alkyle en $C_8$-$C_{30}$,
- les oligomères homo et copolymères d'esters vinyliques ayant des groupements alkyles en $C_8$-$C_{30}$,
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,
- les esters et les polyesters, et
- leurs mélanges.

Selon un mode préféré de l'invention, le ou les beurres particuliers sont d'origine végétale tels que ceux décrit dans Ullmann's Encyclopedia of Industrial Chemistry (« Fats and Fatty Oils», A. Thomas, publié le 15/06/2000, D01: 10.1002/14356007.a10_173, point 13.2.2.2. Shea Butter, Borneo Tallow, and Related Fats (Vegetable Butters)).

On peut citer plus particulièrement les triglycérides en C10-C18 (nom INCI : C10-18 Triglycerides) comportant à la température de 25°C et à pression atmosphérique (760 mm Hg) une fraction liquide et une fraction solide, le beurre de karité, le beurre de Karité Nilotica (*Butyrospermum parkii*), le beurre de Galam, (*Butyrospermum parkii*), le beurre ou graisse de Bornéo ou tengkawang tallow) (*Shorea stenoptera*), beurre de Shorea, beurre d'Illipé, beurre de Madhuca ou Bassia Madhuca longifolia, beurre de mowrah (*Madhuca Latifolia*), beurre de Katiau (*Madhuca mottleyana*), le beurre de Phulwara (*M. butyracea*), le beurre de mangue (*Mangifera indica*), le beurre de Murumuru (*Astrocatyum murumuru*), le beurre de Kokum (*Garcinia Indica*), le beurre d'Ucuuba (*Virola sebifera*), le beurre de Tucuma, le beurre de Painya (Kpangnan) (*Pentadesma butyracea*), le beurre de café (*Coffea arabica*), le beurre d'abricot (*Prunus Armeniaca*), le beurre de Macadamia (*Macadamia Temifolia*), le beurre de pépin de raisin (*Vitis vinifera*), le beurre d'avocat (*Persea gratissima*), le beurre d'olives (*Olea europaea*), le beurre d'amande douce (*Prunus amygdalus dulcis*), le beurre de cacao (*Theobroma cacao*) et le beurre de tournesol, le beurre sous le nom INCI Astrocaryum Murumuru Seed Butter, le beurre sous le nom INCI Theobroma Grandiflorum Seed Butter, et le beurre sous le nom INCI Irvingia Gabonensis Kernel Butter, les esters de jojoba (mélange de cire et d'huile de jojoba hydrogénée)(nom INCI : Jojoba esters) et les esters éthyliques de beurre de karité (nom INCI : Shea butter ethyl esters), et leurs mélanges.

De préférence, l'agent gélifiant est choisi parmi les palmitates de dextrine.

Avantageusement, un agent gélifiant de phase grasse selon l'invention est un agent gélifiant thermosensible, à savoir qui réagit à la chaleur, et notamment est un agent gélifiant solide à température ambiante et liquide à une

température supérieure à 40°C, de préférence supérieure à 50°C.

Avantageusement, un agent gélifiant de phase grasse selon l'invention est un agent gélifiant thixotrope ou apte à conférer à la solution qui le comprend un comportement thixotrope.

Un tel agent gélifiant thixotrope est notamment choisi parmi les silices pyrogénées éventuellement traitées hydrophobes, décrites précédemment.

Selon un mode de réalisation, une dispersion selon l'invention peut comprendre de 0,1% à 75%, de préférence de 0,5% à 60%, en particulier de 1% à 40%, mieux de 1,5% à 20%, et préférentiellement de 1% à 4%, en poids d'agent(s) gélifiant(s) par rapport au poids total de la dispersion.

Selon l'invention, une dispersion selon l'invention peut comprendre de 0,5% à 99,99%, de préférence de 1% à 70%, en particulier de 1,5% à 50%, mieux de 2% à 40%, en particulier de 2,5% à 30%, et préférentiellement de 10 % à 20%, en poids d'agent(s) gélifiant(s) par rapport au poids total de la phase grasse.

*Huile(s)*

Selon l'invention, la phase grasse d'une dispersion selon l'invention peut en outre comprendre au moins une huile H1, de préférence dans laquelle le polymère cationique est soluble. L'huile H1 correspond donc avantageusement à un bon solvant du polymère cationique.

Les dispersions selon l'invention peuvent comprendre une seule huile H1 ou un mélange de plusieurs huiles H1. Une dispersion selon l'invention peut donc comprendre au moins une, au moins deux, au moins trois, au moins quatre, au moins cinq, voire plus, d'huile(s) H1 telle(s) que décrite(s) ci-après.

On entend par « huile » un corps gras liquide à la température ambiante (25°C).

Comme huiles H1 utilisables dans la composition de l'invention, on peut citer par exemple :

- les huiles hydrocarbonées d'origine animale, telles que le perhydrosqualène et le squalane ;
- les esters et les éthers de synthèse, notamment d'acides gras, comme les huiles de formules $R_1COOR_2$ et $R_1OR_2$ dans laquelle $R_1$ représente le reste d'un acide gras en $C_8$ à $C_{29}$, et $R_2$ représente une chaîne hydrocarbonée, ramifiée ou non, en $C_3$ à $C_{30}$, comme par exemple l'huile de Purcellin, l'isononanoate d'isononyle, le néopentanoate d'isodécyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétrabéhénate de pentaérythrityle (DUB PTB) ou le tétraisostéarate de pentaérythrityle (Prisorine 3631) ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam ;
- les huiles de silicone, comme par exemple les polyméthylsiloxanes (PDMS) volatiles ou non à chaine siliconée linéaire ou cyclique, liquides ou pâteux à température ambiante, notamment les cyclopolydiméthylsiloxanes (cyclométhicones) telles que la cyclohexasiloxane et la cyclopentasiloxane ; les polydiméthylsiloxanes (ou diméthicones) comportant des groupements alkyle, alcoxy ou phényle, pendant ou en bout de chaine siliconée, groupements ayant de 2 à 24 atomes de carbone ; les silicones phénylées comme les phényltriméthicones, les phényldiméthicones, les phényltriméthylsiloxy-diphényl-siloxanes, les diphényl-diméthicones, les diphénylméthyldiphényl trisiloxanes, les 2-phényléthyltriméthyl-siloxysilicates, et les polyméthylphénylsiloxanes ;
- les alcools gras ayant de 8 à 26 atomes de carbone, comme l'alcool cétylique, l'alcool stéarylique et leur mélange (alcool cétylstéarylique), ou encore l'octyldodécanol ;
- les huiles fluorées partiellement hydrocarbonées et/ou siliconées comme celles décrites dans le document JP-A-2-295912 ; et
- leurs mélanges.

Selon un mode de réalisation, l'huile H1 est choisie parmi les esters de formule $R_1COOR_2$, dans laquelle $R_1$ représente le reste d'un acide gras en $C_8$ à $C_{29}$, et $R_2$ représente une chaîne hydrocarbonée, ramifiée ou non, en $C_3$ à $C_{30}$.

Selon un mode de réalisation, l'huile H1 est choisie parmi les alcools gras ayant de 8 à 26 atomes de carbone.

Selon un mode de réalisation, l'huile H1 est choisie parmi les huiles hydrocarbonées ayant de 8 à 16 atomes de carbone, et notamment les alcanes ramifiés en $C_8$-$C_{16}$ (appelées aussi isoparaffines ou isoalcanes), comme l'isododécane (encore appelé 2,2,4,4,6-pentaméthylheptane), l'isodécane, l'isohexadécane, et, par exemple, les huiles vendues sous les noms commerciaux d'Isopars® ou de Permethyls®.

Selon un mode de réalisation préféré, l'huile H1 est choisie dans le groupe constitué de l'isononanoate d'isononyle, de la diméthicone, de l'isohexadécane, du polydiméthylsiloxane, de l'octyldodécanol, du néopentanoate d'isodécyle et de leurs mélanges.

De préférence, l'huile H1 est l'isononanoate d'isononyle.

Selon un mode de réalisation, l'huile H1 n'est pas une huile végétale.

Selon un mode de réalisation, l'huile H1 n'est pas figurée par le polydiméthylsiloxane (PDMS), et de préférence n'est pas une huile de silicone.

Selon un autre mode de réalisation, la phase grasse des gouttes ne comprend pas de polydiméthylsiloxane (PDMS), et de préférence ne comprend pas d'huile de silicone.

Selon un mode de réalisation préféré, une dispersion selon l'invention peut comprendre au moins 1% en poids d'huile(s) H1, de préférence d'isononanoate d'isononyle, par rapport au poids total de ladite composition.

Selon un mode de réalisation, la teneur en huile(s) H1 dans la phase grasse est comprise entre 1% et 99,49%, de préférence entre 20% et 90%, et en particulier entre 30% et 60%, en poids par rapport au poids total de ladite phase grasse.

Selon un mode de réalisation, la phase grasse des dispersions selon l'invention peut comprendre en outre au moins une huile hydrocarbonée d'origine végétale H2. La phase grasse peut comprendre plusieurs huiles H2.

Comme huiles végétales H2, on peut notamment citer les triglycérides liquides d'acides gras en $C_4$-$C_{10}$ comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique (nom INCI : Caprylic/Capric Triglyceride) comme ceux commercialisés par la société Stearineries Dubois ou ceux disponibles sous les dénominations commerciales « Miglyol 810 », « Miglyol 812 » et « Miglyol 818 » par la société Dynamit Nobel, l'huile de jojoba, ou l'huile de beurre de karité.

Parmi les huiles H2, on peut également mentionner les composés suivants : les triglycérides en $C_{10}$-$C_{18}$ liquides à la température ambiante (25°C), les triglycérides d'acides caprylique et caprique, les triglycérides d'acides caprylique, caprique, myristique et stéarique (nom INCI : Caprylic/capric/myristic/stearic Triglyceride), le triéthylhexanoine, l'huile végétale hydrogénée, l'huile de graine de limnanthe *Limnanthes Alba* (nom INCI : Limnanthes Alba (Meadowfoam) Seed Oil), l'huile d'olive *Olea Europaea* (nom INCI : Olea Europaea (Olive) Fruit Oil), l'huile de noix de macadamia (nom INCI : Macadamia Ternifolia Seed Oil), l'huile d'églantier *Rosa Canina* (nom INCI : Rosa Canina Fruit Oil), l'huile de soja (nom INCI : Glycine Soja (Soybean) Oil), l'huile de graines de tournesol (nom INCI : Helianthus Annuus (Sunflower) Seed Oil), l'huile de maïs (nom INCI : Zea Mays (Corn) Oil), l'huile de palme hydrogénée (nom INCI : Hydrogenated Palm Oil), le tribéhénine (nom INCI : tribehenin), le triisostéarine (nom INCI : triisostearin), l'huile de noyau d'abricot (nom INCI : Prunus Armeniaca (Apricot) Kernel Oil), l'huile de son de riz (nom INCI : Oryza Sativa (Rice) Bran Oil), l'huile d'argan (nom INCI : Argania Spinosa Kernel Oil), l'huile d'avocat (nom INCI : Persea Gratissima Oil), l'huile d'onagre (nom INCI : Oenothera Biennis Oil), l'huile de palme (nom INCI : Elaeis Guineensis Oil), l'huile de germe de riz (nom INCI : Oryza Sativa Germ Oil), l'huile de noix de coco hydrogénée (nom INCI : Hydrogenated Coconut Oil), l'huile d'amande douce (nom INCI : Prunus Amygdalus Dulcis Oil), l'huile de pépins de raisin (nom INCI : Vitis Vinifera Seed Oil), l'huile de graine de sésame (nom INCI : Sesamum Indicum Seed Oil), l'huile de graine d'arachide (nom INCI : Arachis Hypogaea Oil), l'huile de colza hydrogénée (nom INCI : Hydrogenated Rapeseed Oil), l'huile de *Mortierella isabellina* (nom INCI : Mortierella Oil), l'huile de graine de carthame (nom INCI : Carthamus Tinctorius Seed Oil), l'huile de noix du Queensland *Macadamia integrifolia* (nom INCI : Macadamia Integrifolia Seed Oil), le tricaprylin (ou triacylglycérol), l'huile végétale (nom INCI : Olus Oil), l'huile de palme extraite du noyau (nom INCI : Elaeis Guineensis Kernel Oil), l'huile de noix de coco (nom INCI : Cocos Nucifera Oil), l'huile de germe de blé (nom INCI : Triticum Vulgare Germ Oil), l'huile de graine de bourrache (nom INCI : Borago Officinalis Seed Oil), l'huile de karité (nom INCI : Butyrospermum Parkii Oil), l'huile de noisette (nom INCI : Corylus Avellana Seed Oil), l'huile de ricin hydrogénée (nom INCI : Hydrogenated Castor Oil), l'huile de noyau de palme hydrogénée (nom INCI : Hydrogenated Palm Kernel Oil), l'huile de graine de mangue (nom INCI : Mangifera Indica Seed Oil), l'huile de graine de grenadier (nom INCI : Punica Granatum Seed Oil), l'huile de graine de chou chinois (nom INCI : Brassica Campestris Seed Oil), l'huile de graine de fruit de la passion (nom INCI : Passiflora Edulis Seed Oil), l'huile de graine de camélia du Japon (nom INCI : Camellia Japonica Seed Oil), l'huile de graine de thé vert (nom INCI : Camellia Sinensis Seed Oil), l'huile de germe de maïs (nom INCI : Zea Mays Germ Oil), l'huile d'hoplostèthe (nom INCI : Hoplostethus Oil), l'huile de noix du Brésil (nom INCI : Bertholletia Excelsa Seed Oil), l'huile de graine de rosier musqué (nom INCI : Rosa Moschata Seed Oil), l'huile de graine d'Inca Inchi (ou Sacha Inchi)(nom INCI : Plukenetia Volubilis Seed Oil), l'huile de graine de Babassu (nom INCI : Orbignya Oleifera Seed Oil), l'huile de graines d'une souche hybride de tournesol (nom INCI : Helianthus Annuus Hybrid Oil), l'huile d'argousier (nom INCI : Hippophae Rhamnoides Oil), l'huile de graine de *Marula* (nom INCI : Sclerocarya Birrea Seed Oil), l'huile de graine de *Aleurites Moluccana* (nom INCI : Aleurites Moluccana Seed Oil), l'huile de graine de rosier rubigineux (nom INCI : Rosa Rubiginosa Seed Oil), l'huile de graine de *Camellia Kissi* (nom INCI : Camellia Kissi Seed Oil), l'huile de graine de baobab (nom INCI : Adansonia Digitata Seed Oil), l'huile de baobab (nom INCI : Adansonia Digitata Oil), l'huile de graine de *Moringa* (nom INCI : Moringa Pterygosperma Seed Oil), l'huile de gaine de périlla (nom INCI : Perilla Ocymoides Seed Oil), l'huile de graine de ricin (nom INCI : Ricinus Communis Seed Oil), l'huile de canola (nom INCI : Canola Oil), l'huile de graine de cassis (nom INCI : Ribes Nigrum Seed Oil), l'huile de graine de thé (nom INCI : Camellia Oleifera Seed Oil), l'huile de graine de framboise (nom INCI : Rubus Idaeus Seed Oil), l'huile de graine de crambe

d'Abyssinie (nom INCI : Crambe Abyssinica Seed Oil), l'huile de graine d'églantier (nom INCI : Rosa Canina Seed Oil), l'huile de vipérine à feuilles de plantain (nom INCI : Echium Plantagineum Seed Oil), l'huile de graine de tomate (nom INCI : Solanum Lycopersicum Seed Oil), l'huile essentielle d'amande amère (nom INCI : Prunus Amygdalus Amara Kernel Oil), l'huile de graine de yuzu (nom INCI : Citrus Junos Seed Oil), l'huile de graine de courge (nom INCI : Cucurbita Pepo Seed Oil), l'huile de vison *Mustela Mustelidae* (nom INCI : Mustela Oil), l'huile de graine de dattier du désert (nom INCI : Balanites Roxburghii Seed Oil), l'huile de graine de *Brassica Napus* (nom INCI : Brassica Napus Seed Oil), l'huile de calophylle (nom INCI : Calophyllum Inophyllum Seed Oil), l'huile de graine de mure arctique (nom INCI : Rubus Chamaemorus Seed Oil), l'huile de graine de pin blanc du Japon (nom INCI : Pinus Pentaphylla Seed Oil), l'huile de graine de pastèque (nom INCI : Citrullus Lanatus Seed Oil), l'huile de graine de noix (nom INCI : Juglans Regia Seed Oil), l'huile de graine de nigelle (nom INCI : Nigella Sativa Seed Oil), l'huile de graine de carotte (nom INCI : Daucus Carota Sativa Seed Oil), l'huile de graine de *Coix Lacryma-jobi Ma-yuen* (nom INCI : Coix Lacryma-jobi Ma-yuen Seed Oil), l'huile de de graine de *Coix Lacryma-jobi* (nom INCI : Coix Lachryma-jobi Seed Oil), le mélange de lipides de la farine de *Triticum Vulgare* (nom INCI : Triticum Vulgare Flour Lipids), le trihydroxyméthoxystéarine (nom INCI : Trihydroxymethoxystearin), le triheptanoine (nom INCI : Triheptanoin), l'huile de graine de canneberge (nom INCI : Vaccinium Macrocarpon Seed Oil), l'huile de vanille (nom INCI : Vanilla Planifolia Fruit Oil), l'huile de graine de canneberge (nom INCI : Oxycoccus Palustris Seed Oil), l'huile d'Açaï (nom INCI : Euterpe Oleracea Fruit Oil), le triester d'huile de ricin hydrogénée et de l'acide isostéarique (nom INCI : Hydrogenated Castor Oil Triisostearate), l'huile de coton hydrogénée (nom INCI : Hydrogenated Cottonseed Oil), l'huile d'olive hydrogénée (nom INCI : Hydrogenated Olive Oil), l'huile d'arachide hydrogénée (nom INCI : Hydrogenated Peanut Oil), l'huile de soja hydrogénée (nom INCI : Hydrogenated Soybean Oil), l'huile extraite de jaune d'œuf de poule (nom INCI : Egg Yolk Oil), l'huile de noyau de noyau de pêche (nom INCI : Prunus Persica Kernel Oil), les glycérides d'huile de canola et de phytostérols (nom INCI : Phytosteryl Canola Glycerides), l'huile de graine de cassissier (nom INCI : Ribes Nigrum (Black Currant) Seed Oil), l'huile de graine de karanja (nom INCI : Pongamia Glabra Seed Oil) et l'huile de roucou (nom INCI : Roucou (Bixa orellana) Oil), l'extrait d'huile d'olive, notamment le phytosqualane, l'huile de rosier muscat, l'huile de coriandre, l'huile de lin, l'huile de chia, l'huile de fénugrec, l'huile de chanvre, et leurs mélanges.

De préférence, l'huile H2 est choisie parmi celles riches en acides gras polyinsaturés.

On entend par "acide gras insaturé" au sens de la présente invention, un acide gras comprenant au moins une double liaison. Il s'agit plus particulièrement d'acides gras à longues chaînes, c'est-à-dire pouvant posséder plus de 14 atomes de carbone. Les acides gras insaturés peuvent être sous forme acide, ou sous forme de sel, comme par exemple leur sel de calcium, ou encore sous forme de dérivés, notamment d'ester(s) d'acide(s) gras.

De préférence, l'huile H2 est choisie parmi les huiles riches en acides gras à longues chaînes, c'est-à-dire pouvant posséder plus de 14 atomes de carbone, et mieux en acides gras insaturés comportant de 18 à 22 atomes de carbone, en particulier en acides gras $\omega$-3 et $\omega$-6. Ainsi, avantageusement, les huiles végétales sont choisies parmi les huiles d'onagre, de bourrache, de pépins de cassis, de chanvre, de noix, de soja, de tournesol, de germes de blé, de fénugrec, de rosier muscat, d'échium, d'argan, de baobab, de son de riz, de sésame, d'amande, de noisette, de chia, de lin, d'olive, d'avocat, de carthame, de coriandre, de colza (notamment Brassica naptus), et leurs mélanges.

De préférence, l'huile H2 est choisie parmi les huiles mates et non brillantes. Peut notamment être citée à ce titre l'huile de Moringa.

Selon un mode de réalisation, la teneur en huile(s) H2 dans la phase grasse d'une dispersion selon l'invention est comprise entre 0% et 40%, de préférence entre 0,1% et 25%, et en particulier entre 1% et 20%, en poids par rapport au poids total de ladite phase grasse.

Selon un mode de réalisation, le ratio massique entre la quantité d'huile(s) H1 et la quantité d'huile(s) H2 va de 0,025 à 99,49, de préférence de 0,8 à 90, et en particulier de 2,5 à 80.

La phase grasse peut comprendre en outre au moins une autre huile différente des huiles H1 et H2.

Une dispersion selon l'invention peut comprendre de 0,0001% à 50%, de préférence de 0,1% à 40%, et mieux de 1% à 25%, en poids d'huile(s) par rapport au poids total de ladite dispersion.

***Composé(s) additionnel(s)***

Selon l'invention, la phase continue aqueuse et/ou la phase grasse dispersée peut/peuvent en outre comprendre au moins un composé additionnel différent des polymères anionique et cationique, de l'agent gélifiant et des huiles susmentionnées.

Les dispersions selon l'invention, et en particulier la phase aqueuse continue et/ou la phase grasse dispersée desdites dispersions, peuvent ainsi en outre comprendre des poudres, des paillettes, des colorants, des agents particulaires insolubles dans la phase grasse, des élastomères de silicone émulsionnants et/ou non émulsionnants, notamment tels que décrit dans EP 2 353 577, des conservateurs, des humectants, des stabilisateurs, des chélateurs, des émollients, des agents modificateurs choisis parmi les agents de texture, de viscosité (par exemple des agents gélifiants/de texture de phase aqueuse différents de la base susmentionnée), de pH, de force osmotique et/ou des modificateurs d'indice de réfraction etc... ou tout additif cosmétique usuel, et leurs mélanges.

Selon un mode de réalisation, les agents particulaires insolubles dans la phase grasse des gouttes sont choisis dans le groupe constitué des pigments, des céramiques, des polymères, notamment des polymères acryliques, et de leurs mélanges.

Les dispersions selon l'invention, et en particulier la phase aqueuse continue et/ou la phase grasse dispersée desdites dispersions, peuvent encore en outre comprendre au moins un actif biologique/cosmétique choisi parmi les agents hydratants, les agents cicatrisants, les agents dépigmentants, les filtres UV, les agents desquamants, les agents antioxydants, les actifs stimulant la synthèse des macromoléculaires dermiques et/ou épidermiques, les agents dermodécontractants, les agents anti-transpirants, les agents apaisants et/ou les agents anti-âge, et leurs mélanges.

*Agents anti-rides ou anti-âge*

A titre représentatif d'agents anti-rides ou anti-âge utilisables dans la présente invention, on peut plus particulièrement mentionner l'adénosine, le rétinol et ses dérivés, l'acide ascorbique et ses dérivés, tels que l'ascorbyl phosphate de magnésium et le glucoside d'ascorbyle ; le tocophérol et ses dérivés, tels que l'acétate de tocophéryle ; l'acide nicotinique et ses précurseurs, tels que la nicotinamide ; l'ubiquinone ; le glutathion et ses précurseurs, tels que l'acide L-2-oxothiazolidine-4-carboxylique ; les composés C-glycoside et leurs dérivés, tels que décrits notamment ci-après ; les extraits de plantes et notamment les extraits de criste marine et de feuille d'olivier, ainsi que les protéines végétales et leurs hydrolysats, tels que les hydrolysats de protéines de riz ou de soja ; ou encore les extraits de graines de Vigna aconitifolia comme ceux commercialisés par la société Cognis sous les références Vitoptine LS9529 et Vit-A-Like LS9737 ; les extraits d'algues et en particulier de laminaires ; les extraits bactériens ; les sapogénines, telles que la diosgénine et les extraits de Dioscorées, en particulier de Wild Yam, en contenant ; les $\alpha$-hydroxyacides ; les $\beta$-hydroxyacides, tels que l'acide salicylique et l'acide n-octanoyl-5-salicylique ; les oligopeptides et pseudodipeptides et leurs dérivés acylés, en particulier l'acide {2-[acetyl-(3-trifluoromethyl-phenyl)-amino]-3-methyl-butyrylamino} acétique et les lipopeptides commercialisés par la société SEDERMA sous les dénominations commerciales Biopeptide CL, Matrixyl 500 et Matrixyl 3000 ; le lycopène ; les sels de manganèse et de magnésium, en particulier les gluconates; l'extrait de graines de seigle sous la dénomintation commerciale Coheliss de Silab , un extrait de feuilles de centella asiatica; les extraits huileux ou aqueux de fruit de vanilla planifolia, l'extrait de fleur de vanilla planifolia ; et leurs mélanges.

*Agents humectants ou hydratants*

Comme agents humectants ou hydratants, on peut citer notamment la glycérine ; la diglycérine ; les glycols, tel que le sorbitol; les bétaïnes ; l'urée et ses dérivés notamment l'Hydrovance® commercialisée par National Starch ; les monosaccharides comme le mannose, les AHA, les BHA, le beta-glucan et en particulier le sodium carboxyméthyl beta-glucane de Mibelle-AG-Biochemistry ; le polyoxybutylène, polyoxyéthylène, ou polyoxypropylène glycérol comme le WILBRIDE S-753L® de NOF corporation ; l'huile de rosier muscat commercialisée par Nestlé ; les sphères de collagène et de chondroitine sulfate d'origine marine (Ateocollagen) commercialisées par la société Engelhard Lyon sous la dénomination sphères de comblement marines ; le niacinamide ; le glycéryl-polyméthacrylate de Sederma vendu sous la dénomination commerciale Lubragel☐MS; la triméthylglycine vendu sous la dénomination commerciale AminocoatD par la société Ashahi Kasei Chemicals ; les extraits de nacre contenant un conchyoline vendus notamment par la compagnie Maruzen (Japon) sous le nom commercial Pearl Extract® ; les extraits de plantes tels qu'un extrait de Castanea sativa ou les extraits aqueux ou huileux de fleur de Camellia japonica et en particulier de la variété alba plena ; des protéines de noisettes hydrolysées ; des polysaccharides de Polyanthes tuberosa ; l'huile de noyau d'Argania spinosa; les homo- et co-polymères de l'acide 2- méthacryloyloxyéthylphosphorylcholine, comme Lipidure HM et Lipidure PBM de NOF; les saccharides tels que le glucose, le fructose, le mannose ou le tréhalose; les glycosaminoglycanes et leurs dérivés tels que l'acide hyaluronique, le hyaluronate de sodium et l'acide hyaluronique acétylé, en particulier des sphères d'acide hyaluronique telles que celles commercialisées par la société Engelhard Lyon; le panthénol; l'allantoïne; l'aloe vera ; les acides aminés libres et leurs dérivés; le glucosamine; l'acide citrique ; les céramides; et leurs mélanges.

*Agents antioxydants*

Comme agents antioxydants, on peut plus particulièrement citer le tocophérol et ses esters, en particulier l'acétate de tocophérol ; l'EDTA, l'acide ascorbique et ses dérivés, en particulier l'ascorbyl magnésium phosphate, l'ascorbyl glucoside et le 3-O-ethyl ascorbic acid ; les chélatants, tels que le BHT, le BHA, le N,N' bis(3,4,5-triméthoxybenzyl) éthylenediamine et ses sels, et leurs mélanges.

*Agents dépigmentants*

Comme agents dépigmentants, on peut citer les céramides, la vitamine C et ses dérivés et notamment la vit CG, CP et 3-O éthyl vitamine C, l'alpha et la béta arbutine, l'acide férulique, l'acide kojique, le résorcinol et ses dérivés et en particulier le 4-butyl resorcinol, l'acide tranexamique et ses dérivés, le D calcium panthéteine sulfonate, l'acide lipoique, l'acide ellagique, la vitamine B3, le phényléthyl résorcinol comme le Symwhite 377® de la société Symrise, une eau de fruit de kiwi (Actinidia chinensis) commercialisée par Gattefosse, un extrait de racine de Paeonia suffructicosa tel que celui commercialisé par la société Ichimaru Pharcos sous la dénomination Botanpi Liquid B®, un extrait de licorice (glycyrrhiza glabra) extract, un extrait de sucre brun (Saccharum officinarum), tel que l'extrait de mélasse commercialisé par la société Taiyo Kagaku sous la dénomination Molasses Liquid, un mélange d'acide undecylenique et phénylalanine undecylenoyl, tel que le Sepiwhite MSH® de Seppic.

Bien entendu, l'homme du métier veillera à choisir les éventuels composé(s) additionnel(s) et/ou leur quantité de telle manière que les propriétés avantageuses de la dispersion selon l'invention ne soient pas ou substantiellement pas altérées par l'adjonction envisagée. En particulier, la nature et/ou la quantité du/des composé(s) additionnel(s) dépend(ent) de la nature aqueuse ou grasse de la phase considérée de la dispersion selon l'invention. Ces ajustements relèvent des compétences de l'homme du métier.

Selon un mode de réalisation, la dispersion selon l'invention comprend de 0,00020% à 10%, de préférence de 0,00025% à 5%, et préférentiellement de 0,0026% à 1% en poids de, et notamment de colorant(s), par rapport au poids total de ladite dispersion.

Parmi les agents conservateurs, on peut notamment citer le phénoxyéthanol, le pentylène glycol et l'EDTA.

Selon un mode de réalisation, les dispersions selon l'invention comprennent au moins un agent conservateur, et de préférence un mélange de plusieurs agents conservateurs.

De préférence, la teneur en poids d'agent(s) conservateur(s) est comprise de 0,01% à 10%, de préférence de 0,5% à 5%, par rapport au poids total de ladite dispersion.

Selon l'invention, une dispersion selon l'invention, et notamment le cœur des gouttes (i.e. la phase grasse), peut également comprendre au moins un agent parfumant.

Parmi les agents parfumants, on peut notamment citer tout type de parfum ou de fragrance, ces termes étant utilisés ici de façon indifférente. Ces parfums ou fragrances sont bien connus de l'homme du métier et incluent notamment ceux mentionnés, par exemple, dans S. Arctander, Perfume and Flavor Chemicals (Montclair, N.J., 1969), S. Arctander, Perfume and Flavor Materials of Natural Origin (Elizabeth, N.J., 1960) et dans "Flavor and Fragrance Materials", 1991 (Allured Publishing Co. Wheaton, III. USA). Les parfums utilisés dans le cadre de la présente invention peuvent comprendre les produits naturels comme les extraits, les huiles essentielles, les absolus, les résinoïdes, les résines, les concrètes, etc... ainsi que les substances basiques de synthèse comme les hydrocarbures, les alcools, les aldéhydes, les cétones, les éthers, les acides, les esters, les acétals, les cétals, les nitriles, etc..., y compris les composés, saturés et insaturés, les composés aliphatiques, alicycliques et hétérocycliques.

La dispersion selon l'invention peut comprendre de 0,01% à 30% en poids d'agent(s) parfumant(s), de préférence de 0,5% à 20% en poids, par rapport au poids total de la dispersion.

Selon un mode de réalisation, les dispersions de l'invention peuvent en outre comprendre de la glycérine. De préférence, une dispersion selon l'invention peut comprendre au moins 5% en poids de glycérine par rapport au poids total de ladite dispersion.

En effet, au-delà de la texture, les dispersions selon l'invention apportent un autre avantage par rapport aux émulsions « classiques » car elles permettent d'utiliser de la glycérine, qui plus est dans des teneurs élevées.

Elles peuvent en particulier comprendre de la glycérine en une teneur supérieure ou égale à 10%, supérieure ou égale à 20%, supérieure ou égale à 30%, supérieure ou égale à 40%, voire jusqu'à 50%, en poids, par rapport au poids total de ladite dispersion.

***Procédé de préparation***

Les dispersions selon l'invention peuvent être préparées par différents procédés.

Ainsi, les dispersions selon l'invention présentent l'avantage de pouvoir être préparées selon un procédé simple « non-microfluidique », à savoir par simple émulsification.

Comme dans une émulsion classique, une solution aqueuse et une solution grasse sont préparées séparément. C'est l'ajout sous agitation de la phase grasse dans la phase aqueuse qui crée l'émulsion directe.

La viscosité de la phase aqueuse peut être maîtrisée, notamment, en jouant sur la quantité de polymère anionique (notamment carbomère) et le pH de la solution. De manière générale, le pH de la phase aqueuse est inférieur à 4,5, ce qui peut impliquer l'ajout d'une troisième solution de soude (BF) dans un dernier temps pour atteindre un pH compris entre 5,5 et 6,5.

La viscosité de la phase aqueuse et la force de cisaillement appliquée au mélange sont les deux principaux

paramètres qui influencent la taille et la monodispersité de l'émulsion.

Les émulsions selon l'invention peuvent également être préparées selon un procédé microfluidique, notamment comme décrit dans les demandes internationales WO 2012/120043 ou WO 2015/055748.

Selon ce mode de réalisation, les gouttes obtenues par ce procédé microfluidique présentent une distribution de taille uniforme.

De préférence, les dispersions de l'invention sont constituées d'une population de gouttes monodispersées, notamment telles qu'elles possèdent un diamètre moyen $\overline{D}$ compris de 1 $\mu$m à 2 500 $\mu$m, voire de 500 $\mu$m à 3000 $\mu$m, et un coefficient de variation Cv inférieur à 10%, voire inférieur à 3%.

Dans le cadre de la présente description, on entend par "gouttes monodispersées" le fait que la population de gouttes de la dispersion selon l'invention possède une distribution de taille uniforme. Des gouttes monodispersées présentent une bonne monodispersité. A l'inverse, des gouttes présentant une mauvaise monodispersité sont dites "polydispersées".

Selon un mode, le diamètre moyen $\overline{D}$ des gouttes est par exemple mesuré par analyse d'une photographie d'un lot constitué de N gouttes, par un logiciel de traitement d'image (Image J). Typiquement, selon cette méthode, le diamètre est mesuré en pixel, puis rapporté en $\mu$m, en fonction de la dimension du récipient contenant les gouttes de la dispersion.

De préférence, la valeur de N est choisie supérieure ou égale à 30, de sorte que cette analyse reflète de manière statistiquement significative la distribution de diamètres des gouttes de ladite émulsion.

On mesure le diamètre Di de chaque goutte, puis on obtient le diamètre moyen $\overline{D}$ en calculant la moyenne arithmétique de ces valeurs :

$$\overline{D} = \frac{1}{N}\sum_{i=1}^{N} D_i$$

A partir de ces valeurs $D_i$, on peut également obtenir l'écart-type $\sigma$ des diamètres des gouttes de la dispersion :

$$\sigma = \sqrt{\frac{\sum_{i=1}^{N}\left(D_i - \overline{D}\right)^2}{N}}$$

L'écart-type $\sigma$ d'une dispersion reflète la répartition des diamètres $D_i$ des gouttes de la dispersion autour du diamètre moyen $\overline{D}$.

En connaissant le diamètre moyen $\overline{D}$ et l'écart-type $\sigma$ d'une dispersion, on peut déterminer que l'on trouve 95,4% de la population de gouttes dans l'intervalle de diamètres [$\overline{D}$-2$\sigma$;$\overline{D}$+2$\sigma$] et que l'on trouve 68,2% de la population dans l'intervalle [$\overline{D}$-$\sigma$; $\overline{D}$+$\sigma$].

Pour caractériser la monodispersité de la dispersion selon ce mode de l'invention, on peut calculer le coefficient de variation :

$$C_v = \frac{\sigma}{\overline{D}}$$

Ce paramètre reflète la répartition des diamètres des gouttes en fonction du diamètre moyen de celles-ci.

Le coefficient de variation Cv des diamètres des gouttes selon ce mode de l'invention est inférieur à 10%, de préférence inférieur à 5%, voire inférieur à 3%.

Alternativement, la monodispersité peut être mise en évidence en plaçant un échantillon de dispersion dans un flacon à section circulaire constante. Une agitation douce par rotation de un quart de tour sur une demi-seconde autour de l'axe de symétrie traversant le flacon, suivie d'un repos d'une demi-seconde est effectuée, avant de répéter l'opération en sens inverse, et ce quatre fois de suite.

Les gouttes de la phase dispersée s'organisent sous une forme cristalline lorsqu'elles sont monodispersées. Ainsi, elles présentent un empilement suivant un motif se répétant suivant dans les trois dimensions. Il est alors possible d'observer, un empilement régulier qui indique une bonne monodispersité, un empilement irrégulier traduisant la polydispersité de la dispersion.

Pour obtenir des gouttes monodisperses, on peut également mettre en œuvre la technique de la microfluidique

(Utada et al. MRS Bulletin 32, 702-708 (2007) ; Cramer et al. Chem. Eng. Sci. 59, 15, 3045-3058 (2004)), et plus particulièrement des dispositifs microfluidiques de type co-flow (les fluides vont dans la même direction) ou flow-focusing (les fluides vont dans des directions différentes, et typiquement dans des sens opposés).

La présence, dans la phase grasse, d'agent(s) gélifiant(s), telle qu'envisagée précédemment, peut nécessiter des ajustements au niveau du procédé de préparation d'une dispersion selon l'invention. En particulier, le procédé de préparation d'une telle dispersion selon l'invention peut comprendre une étape de chauffage (entre 40°C et 150°C, notamment entre 50°C et 90°C) de la phase grasse avant mélange/mise en contact de ladite phase grasse avec la phase aqueuse et, le cas échéant dans le cas d'un procédé « non-microfluidique » tel que susmentionné, le maintien de ce chauffage lors de l'agitation jusqu'à l'obtention de l'émulsion recherchée. Dans le cas d'un procédé « microfluidique » tel que susmentionné, cette étape de chauffage a lieu au moins au niveau de la phase grasse et du dispositif microfluidique lors de la fabrication de la dispersion.

Selon un mode de réalisation, le procédé de préparation des dispersions de l'invention comprend une étape de formation des gouttes comprenant :

- la mise en contact d'un fluide aqueux FE et d'un fluide huileux FI tels que définis ci-dessus; et
- la formation des gouttes de phase grasse, constituée du fluide huileux FI, dispersée dans une phase aqueuse continue, constituée de fluide FE, lesdites gouttes comprenant une écorce isolant le cœur des gouttes de la phase grasse de la dispersion.

Selon un mode de réalisation, le fluide FI est initialement préparé en mélangeant une phase grasse destinée à former le cœur des gouttes, au moins un premier polymère précurseur de la coacervation tel qu'un polymère cationique tel que défini précédemment, au moins un agent gélifiant et en outre, de façon optionnelle, au moins une huile et/ou au moins un composé additionnel tel(s) que susmentionné(s).

Selon un mode de réalisation, le fluide FE est initialement préparé en mélangeant une phase aqueuse destinée à former la phase continue de la dispersion, au moins un deuxième polymère précurseur de la coacervation, tel qu'un polymère anionique tel que défini précédemment et en outre, de façon optionnelle, au moins une base, un composé additionnel, des conservateurs et/ou autres produits solubles dans l'eau tels que par exemple la glycérine.

Selon un mode de réalisation, le polymère cationique initialement présent dans le fluide huileux FI sert notamment à la formation de l'écorce des gouttes.

Selon un mode de réalisation, la phase continue aqueuse de la dispersion formée comprend, voire est figurée par, la phase aqueuse du fluide FE. Le polymère anionique initialement présent dans le fluide FE sert notamment à la formation de l'écorce des gouttes. Ledit polymère anionique contribue en outre à augmenter la viscosité du fluide FE, et donc de la phase continue aqueuse.

Selon un mode de réalisation, l'étape de formation des gouttes peut en outre comprendre une étape d'injection d'une solution d'augmentation de la viscosité de la phase aqueuse continue du fluide FE. De préférence, la solution d'augmentation de la viscosité est aqueuse. Cette solution d'augmentation de la viscosité est typiquement injectée dans le fluide externe aqueux FE après formation de la dispersion selon l'invention, et donc après formation des gouttes.

Selon un mode de réalisation, la solution d'augmentation de la viscosité comprend une base, notamment un hydroxyde d'alcalin, tel que l'hydroxyde de sodium.

Selon un mode de réalisation, le procédé de préparation d'une dispersion selon l'invention comprend une étape de chauffage du fluide huileux FI, comprenant la phase grasse de la dispersion, à une température comprise de 40°C et 150°C, de préférence de 50°C à 90°C, préalablement à l'étape susmentionnée de formation des gouttes, et donc avant mélange/mise en contact de ladite phase grasse avec la phase aqueuse. Dans le cas d'un procédé « non-microfluidique » tel que susmentionné, cette étape de chauffage peut être maintenue lors de l'agitation permettant l'obtention de l'émulsion recherchée. Dans le cas d'un procédé « microfluidique » tel que susmentionné, cette étape de chauffage a lieu au moins au niveau de la phase grasse et du dispositif microfluidique lors de la fabrication de la dispersion.

Selon un mode de réalisation, la température de l'étape de chauffage est comprise de 50°C à 90°C, voire de 50°C à 80°C, de préférence de 50°C à 70°C, et plus préférentiellement de 55°C à 70°C, voire de 55°C à 65°C.

Selon un mode de réalisation, lorsque le fluide huileux FI comprend de 5% à 15% en poids d'agent(s) gélifiant(s), en particulier thermosensible(s), par rapport au poids total dudit fluide huileux FI, ledit fluide huileux FI est chauffé à une température de 65 à 70°C.

Selon un mode de réalisation, lorsque le fluide huileux FI comprend de 15% à 99,99%, de préférence de 15% à 40%, en poids d'agent(s) gélifiant(s) par rapport au poids total dudit fluide huileux FI, ledit fluide huileux FI est chauffé à une température de 80 à 90°C.

Selon ce mode de réalisation, le procédé de préparation des dispersions de l'invention comprend les étapes suivantes :

- optionnellement, le chauffage du fluide huileux FI tel que décrit ci-dessus, à une température comprise de 40°C et

150°C, de préférence de 50°C à 90°C ;

- la mise en contact du fluide aqueux FE tel que décrit ci-dessus et du fluide huileux FI ; et
- la formation des gouttes de phase grasse, constituée du fluide huileux FI, dispersée dans une phase aqueuse continue constituée de fluide FE, lesdites gouttes comprenant une écorce isolant le cœur des gouttes de la phase grasse de la dispersion.

Avantageusement, la présence d'un agent gélifiant dans le fluide huileux FI permet de s'affranchir de l'utilisation d'un fluide intermédiaire tel que décrit dans la demande WO 2012/120043. Cela ressort notamment de l'exemple 1 ci-après. En cela, le procédé de préparation d'une dispersion selon l'invention est simplifié par rapport au procédé de préparation décrit dans WO 2012/120043.

***Utilisations***

De manière préférée, une dispersion selon l'invention est directement utilisable, à l'issue des procédés de préparation précités, à titre de composition, notamment cosmétique. La dispersion selon l'invention est également utilisable à titre de composition, notamment cosmétique. Ainsi, selon un mode de réalisation particulier, la dispersion selon l'invention, lorsque préparée au moyen d'un procédé micro-fluidique tel que décrit ci-dessus, est utilisable à titre de composition après séparation des gouttes et redispersion de celles-ci dans une seconde phase appropriée.

Les dispersions ou compositions selon l'invention peuvent notamment être utilisées dans le domaine cosmétique.

L'invention concerne ainsi également l'utilisation d'une dispersion selon l'invention pour la préparation d'une composition, notamment cosmétique.

Les dispersions ou compositions selon l'invention peuvent comprendre, outre les ingrédients susmentionnés, au moins un milieu physiologiquement acceptable.

La présente invention concerne ainsi également une composition, notamment cosmétique, comprenant au moins une dispersion selon l'invention, en association avec un milieu physiologiquement acceptable.

Dans le cadre de l'invention, et sauf mention contraire, on entend par "milieu physiologiquement acceptable", un milieu approprié aux applications cosmétiques, et convenant notamment à l'application d'une composition de l'invention sur une matière kératinique, notamment la peau et/ou les cheveux, et plus particulièrement la peau.

Le milieu physiologiquement acceptable est généralement adapté à la nature du support sur lequel doit être appliquée la composition, ainsi qu'à l'aspect sous lequel la composition doit être conditionnée.

Selon un mode de réalisation, le milieu physiologiquement acceptable est figuré directement par la phase continue aqueuse telle que décrite ci-dessus.

Les compositions cosmétiques de l'invention peuvent être par exemple une crème, une émulsion, une lotion, un sérum, un gel et une huile pour la peau (mains, visage, pieds, etc.), un fond de teint (liquide, pâte) une préparation pour bains et douches (sels, mousses, huiles, gels, etc.), un produit de soins capillaires (teintures capillaires et décolorants), un produit de nettoyage (lotions, poudres, shampoings), un produit d'entretien pour la chevelure (lotions, crèmes, huiles), un produit de coiffage (lotions, laques, brillantines), un produit pour le rasage (savons, mousses, lotions, etc.), un produit destiné à être appliqué sur les lèvres , un produit solaire, un produit de bronzage sans soleil, un produit permettant de blanchir la peau, un produit antirides. En particulier, les compositions cosmétiques de l'invention peuvent être un sérum anti-âge, un sérum jeunesse, un sérum hydratant ou une eau parfumée.

La présente invention concerne également un procédé non thérapeutique de traitement cosmétique d'une matière kératinique, notamment la peau et/ou les cheveux, et plus particulièrement la peau, comprenant une étape d'application sur ladite matière kératinique d'au moins une dispersion ou d'au moins une couche d'une composition cosmétique susmentionnée.

Les dispersions de l'invention, en particulier l'écorce des gouttes desdites dispersions, présentent avantageusement des résistances mécaniques améliorées par rapport à ces mêmes dispersions mais dénuées de l'agent gélifiant dans la phase grasse dispersée (à l'image par exemple des dispersions décrites dans le document WO 2012/120043).

Cette résistance mécanique améliorée permet d'éviter des cisaillements ou des fragmentations des gouttes lors du transport des dispersions ou des produits cosmétiques les contenant.

De plus, cette amélioration de la résistance mécanique permet avantageusement de pouvoir conditionner les dispersions et les produits cosmétiques les comprenant dans des packagings pouvant comprendre de l'air sans risque de cisaillements et/ou de fragmentations.

En outre, les dispersions selon l'invention présentent avantageusement des propriétés de stabilité similaires à des dispersions identiques dépourvues d'agent(s) gélifiant(s) dans la phase grasse, à l'image par exemple des dispersions décrites dans le document WO 2012/120043.

Les dispersions de gouttes dispersées selon l'invention présentent avantageusement des viscosités compatibles avec une manipulation aisée du produit obtenu.

Dans toute la description, y compris les revendications, l'expression « comprenant un » doit être comprise comme étant synonyme de « comprenant au moins un », sauf si le contraire est spécifié.

Les expressions « compris entre ... et ... », « compris de ... à ... » et « allant de ... à ... » doivent se comprendre bornes incluses, sauf si le contraire est spécifié.

Les quantités des ingrédients figurant dans les exemples sont exprimées en pourcentage en poids par rapport au poids total de la composition, sauf indication contraire.

Les exemples qui suivent illustrent la présente invention sans en limiter la portée.

## EXEMPLES

Matériel

| Equipement | Utilisation |
|---|---|
| 2Ppousses seringues | Alimentation du dispositif microfluidique |
| Pompes péristaltiques | |
| Seringues en verre de 50mL | |
| Seringue en verre de 25 mL | |
| Chauffe seringue | |
| 3 vannes 3 voies | |
| Capillaire en PTFE | |
| 1 device | Dispositif microfluidique |
| 1 tour centrale | Support de l'alimentation |
| 1 statif | Support du dispositif microfluidique |
| Moteur d'agitation | Préparation des solutions |
| Plaque magnétique chauffante thermostatée | |
| Balance | |
| Consommables de laboratoire | Utilisation régulière |

Sauf indication contraire, les compositions décrites ci-après résultent d'un procédé microfluidique, notamment tel que décrit dans la description ou dans WO/2010/063937.

**Exemple 1** : **Préparation d'une dispersion de gouttes avec ou sans utilisation de fluide intermédiaire**

Cet exemple a consisté à préparer une dispersion par un procédé réalisé en présence d'une phase intermédiaire appelée MF (exemple 1A comparatif) ou en son absence (exemple 1B selon l'invention).

Les compositions des phases (fluides) permettant la préparation des dispersions 1A et 1B sont les suivantes :

| | | | **1A (comparatif)** | **1B (invention)** |
|---|---|---|---|---|
| **Fluide** | **Nom** | **INCI** | **%w/w** | **%w/w** |
| **IF** | Lanol 99 | Isononyl Isononanoate | QSP* | QSP* |
| | Rheopearl KL2 | Dextrin Palmitate | 0,5/5/10 | 0,5/5/10 |
| | KF 8004 | Amodimethicone | 0,5 | 0,5 |
| **MF** | Lanol 99 | Isononyl Isononanoate | 100 | - |
| **OF** | Eau Osmosée | - | 83,34 | 83,34 |
| *($\mu$= 811 pH = 3,48)* | Glycerol | Glycerin | 7,00 | 7,00 |

(suite)

| | | | 1A (comparatif) | 1B (invention) |
|---|---|---|---|---|
| **Fluide** | **Nom** | **INCI** | **%w/w** | **%w/w** |
| | Zemea | Propanediol | 6,00 | 6,00 |
| | Microcare PTG | Pentylèneglycol | 2,35 | 2,35 |
| | Microcare PE | Phenoxyethanol | 0,95 | 0,95 |
| | Carbomer Tego 340 FD | Carbomer | 0,24 | 0,24 |
| | Rhodicare T | Xanthan | 0,12 | 0,12 |
| Base | Eau Osmosée | Aqua | 99,7 | 99,7 |
| | NaOH | Sodium Hydroxyde | 0,3 | 0,3 |
| ** QSP : quantité suffisante pour* | | | | |

Protocole de préparation

Pour l'OF :

- Le carbomère est dispersé dans l'eau osmosée et mis sous agitation pendant 2 heures à l'aide d'une pâle défloculeuse.
- La glycérine, le Propanediol (Zemea) ainsi que le xanthane sont par la suite ajoutés. Suite à ces ajouts, le mélange est placé sous agitation pendant 10 min.
- Le Phénoxyéthanol (Microcare PE) et le Pentylèneglycol (Microcare PTG) sont ajoutés. Le mélange est agité pendant 5 min.
- La soude est ensuite ajoutée.
- La dernière étape consiste à mélanger la solution pendant 1 heure.

Pour la base : La soude et l'eau sont mélangées à l'aide d'un barreau magnétique pendant 5 min.

Pour l'IF :

- L'amodiméthicone est ajoutée au Lanol 99 puis mélangée à l'aide d'un barreau magnétique pendant 5 min.
- On chauffe le mélange à 80°C puis on ajoute le Rheopearl KL2 sous agitation magnétique.
- Ce mélange peut ensuite être placé dans un bain marie chauffé à 75°C sous agitation magnétique pendant 1 heure.

La solution d'IF chauffée à 75°C est introduite dans une seringue reliée à un chauffage permettant de maintenir la solution à chaud. Pour réduire les pertes thermiques le dispositif microfluidique a été installé directement en sortie de seringue. La quantité d'agent gélifiant a été modifiée : 0,5%, 5% et 10% en poids par rapport au poids total de la phase IF.

Pour stabiliser le système, le montage a été utilisé une heure après l'introduction de la solution d'IF dans la seringue.

Exemple 1A :

Dans cet essai 1A (réalisé en présence d'une phase intermédiaire MF), les débits suivants ont été utilisés :

| | |
|---|---|
| OF | 240 mL/hr |

(suite)

| | |
|---|---|
| MF | 5 mL/hr |
| IF | 15 mL/hr |
| Base | 28,8 mL/hr |

La présence du gélifiant Rheopearl KL2 a conduit à une opacification des gouttes de la dispersion. De plus, quelle que soit la concentration en agent gélifiant, l'aspect des gouttes obtenues ne s'est pas avéré uniforme. Il a été démontré que selon ce procédé, le gélifiant n'a pas diffusé à travers la MF et n'a pas gélifié la phase grasse dans sa totalité.

Exemple 1B :

Un autre essai 1B a été réalisé avec l'IF contenant 10% de Rheopearl KL2 et sans phase intermédiaire MF, avec les débits suivants :

| | |
|---|---|
| **OF** | 240 mL/hr |
| **MF** | 0 mL/hr |
| **IF** | 20 mL/hr |
| **Base** | 28,8 mL/hr |

En mettant en œuvre un procédé dépourvu de phase intermédiaire, il a été montré que les gouttes de la dispersion finale ont un aspect entièrement uniforme.

La stabilité dans le temps du système de l'essai 1B sans MF (i.e. selon l'invention) a été étudiée. Pour cela, une production de la dispersion de gouttes a été réalisée dans les mêmes conditions que l'essai 1A et le diamètre des gouttes a été mesuré au cours du temps. Un diamètre identique tout le long de la manipulation permet de s'assurer qu'aucun paramètre n'a été modifié. En effet, un encrassage de la buse ou un changement de viscosité de l'IF aurait automatiquement été détecté par une modification de la taille des bulles. Les résultats obtenus sont les suivants :

Tableau 1 : Mesure des diamètres durant la production de dispersion comprenant des gouttes gélifiées

| **Temps (min)** | 5 | 30 | 60 | 120 | 180 | 240 | 300 |
|---|---|---|---|---|---|---|---|
| **Diamètre moyen* (mm)** | 1,221 | 1,200 | 1,192 | 1,211 | 1,213 | 1,222 | 1,249 |
| **Covariance (%)** | 4,471 | 2,895 | 2,885 | 2,471 | 2,046 | 2,586 | 2,852 |
| **Mesures effectuées sur 100 gouttes* | | | | | | | |

La covariance des diamètres moyens au cours du temps est de 1,07%. La différence de diamètre est négligeable.

Le procédé microfluidique utilisé sans phase intermédiaire MF est donc stable. On peut supposer que la présence du gélifiant huileux ralentit la diffusion de l'amodiméthicone à l'interface eau/huile et empêche l'encrassement de la buse. L'utilisation d'une phase intermédiaire n'est donc pas nécessaire contrairement à l'obtention d'une dispersion par microfluidique avec la composition 1A comparative.

**Exemple 2 : Résistance mécanique des dispersions de gouttes comprenant un agent gélifiant**

2.1. Test de transport

En ce qui concerne la résistance mécanique des dispersions selon l'invention, un test de transport a été réalisé sur un échantillon sérum avec 20% de Rheopearl KL2 dans la phase huileuse. A cet effet, un réceptacle classique de 100 mL ne requérant pas une atmosphère spécifique dénuée d'air est rempli à 50% avec l'échantillon.

| Nom commercial | Nom INCI | Pourcentage dans la phase (%m) | Pourcentage massique final (%m) |
|---|---|---|---|
| **Phase aqueuse** | | | |
| Eau osmosée | Aqua | 86,09% | 78,80% |
| Glycérine | Glycerin | 5,80% | 5,31% |
| Zemea | Butylene glycol | 5,00% | 4,58% |
| Microcare PTG | Pentylenglycol | 2,00% | 1,83% |
| Rhodicare T | Xanthan gum | 0,10% | 0,09% |
| Microcare PE | Phenoxyethanol | 0,79% | 0,72% |
| Carbomer Tego 340 FD | carbomer | 0,20% | 0,18% |
| Sodium hydroxyde pellets PRS codex | Sodium hydroxyde | 0,03% | 0,03% |
| *Total* | | 100,00% | 91,54% |
| **Phase grasse** | | | |
| Lanol 99 | Isononyl isononanoate | 79,10% | 6,69% |
| Rheopearl KL2 | Dextrin palmitate | 20,00% | 1,69% |
| KF 8004 | Amodimethicone | 0,50% | 0,04% |
| Phat Blue DC 6204 | CI 61565 / CI 60725 | 0,40% | 0,03% |
| *Total* | | 100,00% | 8,458% |
| **TOTAL** | | | 100,00% |

Ce test de transport consiste en un aller-retour Marseille-Paris, par la Poste.

Il a été observé que les gouttes de ladite dispersion, soumises à ce test de transport, ne se sont pas fragmentées : elles sont donc restées intactes malgré le recours à un packaging non « airless ».

2.2. Test de roulement

Un test de roulement a été effectué sur les deux échantillons différents suivants :

- 1$^{er}$ échantillon = dispersion 2A : gouttes gélifiées avec 10% de Rheopearl KL2 dans l'IF (selon l'invention)
- 2$^{ème}$ échantillon = dispersion 2B : gouttes non gélifiées (sans Rheopearl KL2) dans l'IF (comparatif)

Composition du 1$^{er}$ échantillon :

| Nom commercial | Nom INCI | %m dans la phase | %m finale |
|---|---|---|---|
| **PHASE AQUEUSE** | | | |
| Eau osmosée | *Aqua* | QSP* | QSP* |
| Glycerine | *Glycerine* | 16,152 | 14,844 |
| Zemea | *Propanediol* | 5,384 | 4,948 |
| Butylène glycol | *Butylene glycol* | 5,384 | 4,948 |
| Microcare PTG | *Pentylenglycol* | 2,146 | 1,972 |
| Microcare PE | *Phenoxyethanol* | 0,858 | 0,789 |
| Carbomer Tego 340FD | *Carbomer* | 0,233 | 0,214 |

(suite)

| Nom commercial | Nom INCI | %m dans la phase | %m finale |
|---|---|---|---|
| **PHASE AQUEUSE** | | | |
| EDETA | *Disodium EDTA* | 0,037 | 0,034 |
| Sodium hydroxyde pellets PRS codex | *Sodium hydroxyde* | 0,043 | 0,039 |
| ***Total*** | | *100* | *91,900* |
| **PHASE GRASSE** | | | |
| DUB ININ | *Isononyl isononanoate* | QSP* | QSP* |
| **Rheopearl KL2** | ***Dextrin palmitate*** | **10** | **0,810** |
| KF 8004 | *Amodiméthicone* | 0,5 | 0,041 |
| Phat Blue DC 6204 | *CI 61565, CI 60725* | 0,01 | <0,01 |
| ***Total*** | | 100 | *8,100* |
| **Total** | | | **100,00** |
| ** QSP : quantité suffisante pour* | | | |

Composition du 2ème échantillon :

| Nom commercial | Nom INCI | %m dans la phase | %m finale |
|---|---|---|---|
| **PHASE AQUEUSE** | | | |
| Eau osmosée | *Aqua* | QSP* | QSP* |
| Glycérine | *Glycerine* | 16,152 | 14,844 |
| Zemea | *Propanediol* | 5,384 | 4,948 |
| Butylène glycol | *Butylene glycol* | 5,384 | 4,948 |
| Microcare PTG | *Pentylenglycol* | 2,146 | 1,972 |
| Microcare PE | *Phenoxyethanol* | 0,858 | 0,789 |
| Carbomer Tego 340FD | *Carbomer* | 0,233 | 0,214 |
| EDETA | *Disodium EDTA* | 0,037 | 0,034 |
| Sodium hydroxyde pellets PRS codex | *Sodium hydroxyde* | 0,043 | 0,039 |
| ***Total*** | | *100* | *91,900* |
| **PHASE GRASSE** | | | |
| DUB ININ | *Isononyl isononanoate* | QSP* | QSP* |
| KF 8004 | *Amodiméthicone* | 0,5 | 0,041 |
| Phat Blue DC 6204 | *CI 61565, CI 60725* | 0,01 | <0,01 |
| ***Total*** | | 100 | *8,100* |
| **Total** | | | **100,00** |
| ** QSP : quantité suffisante pour* | | | |

*Mode opératoire du test de roulement*

Sur un pot en verre de 8,5 cm de haut et 4 cm de diamètre, une ligne est tracée à 4 cm de haut. Pour les deux échantillons, les gouttes ont été collectées jusqu'à cette ligne limite. Chaque pot a ensuite été placé à température ambiante pendant 1 semaine.

Après ce laps de temps, chaque pot a été délicatement incliné à l'horizontale et laissé au repos pendant une heure. Ils ont ensuite été déposés sur les rouleaux d'un roller-mixer. Les différents cycles de roulement ont été imposés suivant l'ordre ci-dessous :

- 6 tpm pendant 3 min
- 20 tpm pendant 3 min
- 30 tpm pendant 3 min
- 30 tpm pendant 1 heure
  tpm = tours par minute

Une observation visuelle des deux échantillons a été effectuée pendant le cisaillement pour décrire l'élasticité de la membrane. Une autre observation a également été réalisée entre chaque cycle pour étudier la fragmentation des bulles.

*Observations*

| Protocole | Dispersion 2B ne comprenant pas d'agent gélifiant (comparatif) | Dispersion 2A comprenant un agent gélifiant (invention) |
|---|---|---|
| 6 tpm pendant 3 min | Aucune fragmentation | Aucune fragmentation |
| 20 tpm pendant 3 min | Aucune fragmentation | Aucune fragmentation |
| 30 tpm pendant 3 min | Petite fragmentation + déformation des gouttes. Certaines gouttes ont un comportement :<br>- déformation irréversible<br>- déformation réversible | Aucune fragmentation |
| 30 tpm pendant 1 h | Forte fragmentation au fur et à mesure du temps<br>Pendant 25 min les gouttes se déforment et se fragmentent Après 25 min : les gouttes se fragmentent immédiatement en multiples fines gouttelettes Au bout de 40 min : On a du mal à distinguer les gouttes. Les gouttes sont presque quasiment détruites. | Aucune fragmentation<br>Les gouttes restent intactes durant 1 heure |

Il a ainsi été démontré que la dispersion 2A selon l'invention présente une meilleure résistance mécanique qu'une même dispersion 2B dépourvue d'agent gélifiant.

2.3. Test de la bille

*Protocole*

Une bille en inox a été placée dans un vial de 12 ml contenant une composition et le comportement des gouttes au passage de la bille a été étudié.

Deux vials sont ainsi préparés, comprenant respectivement les dispersions 2A et 2B décrites dans le test de roulement 2.2 ci-dessus.

*Observations*

Le passage de la bille sur la composition 2B (comparatif) ne comprenant pas d'agent gélifiant conduit à quelques fragmentations des gouttes non gélifiées. La composition 2A (selon l'invention) contenant des gouttes comprenant un agent gélifiant se trouve être très résistante au passage de la bille. Aucune déformation ou fragmentation n'a été observée pour la composition 2A. Après plusieurs passages de la bille, l'échantillon non gélifié 2B présente une forte fragmentation

que nous n'observons pas pour l'échantillon 2A avec les gouttes gélifiées.

**Exemple 3** : **Préparation d'un sérum de soin**

La composition 3A (selon l'invention) est une composition dont les gouttes de phase grasse comprennent un agent gélifiant. Elle a été préparée à partir des compositions de phases suivantes (cf. tableau ci-dessous) :

***Composition 3A***

| **Fluide** | **Nom** | **INCI** | **%w/w** |
|---|---|---|---|
| OF | Eau Osmosée | - | 67,63 |
| | Glycerol | Glycérine | 17,31 |
| | Zemea | Propanediol | 5,77 |
| *($\mu$ = 552 mPa.s pH = 4,60)* | Butylène Glycol | Butylène glycol | 5,77 |
| | Microcare PTG | Pentylèneglycol | 2,3 |
| | Microcare PE | Phénoxyéthanol | 0,92 |
| | Carbomer Tego 340 FD | Carbomer | 0,25 |
| | EDETA | Disodium EDTA | 0,04 |
| | NaOH | Hydroxyde de sodium | 0,01 |
| Base | Eau Osmosée | Eau | 99,5 |
| | NaOH | Hydroxyde de sodium | 0,5 |
| IF | Lanol 99 | Isononyl Isononanoate | 89,35 |
| (gélifiée) | **Rheopearl KL2** | Dextrin Palmitate | 10 |
| | KF 8004 | Amodimethicone | 0,5 |
| | Phat Black DC 9206 | CI 61565, CI 60725, CI 26100 | 0,15 |

La composition 3B (comparative) est une composition dont les gouttes de phase grasse ne comprennent pas d'agent gélifiant. Elle a été préparée à partir des compositions de phases suivantes (cf. tableau ci-dessous) :

***Composition 3B***

| **Fluide** | **Nom** | **INCI** | **%w/w** |
|---|---|---|---|
| OF | Eau Osmosée | - | 67,63 |
| | Glycerol | Glycérine | 17,31 |
| | Zemea | Propanediol | 5,77 |
| *($\mu$ = 552 mPa.s pH = 4,60)* | Butylène Glycol | Butylène glycol | 5,77 |
| | Microcare PTG | Pentylèneglycol | 2,3 |
| | Microcare PE | Phénoxyéthanol | 0,92 |
| | Carbomer Tego 340 FD | Carbomer | 0,25 |
| | EDETA | Disodium EDTA | 0,04 |
| | NaOH | Hydroxyde de sodium | 0,01 |
| Base | Eau Osmosée | Eau | 99,5 |
| | NaOH | Hydroxyde de sodium | 0,5 |

(suite)

| Fluide | Nom | INCI | %w/w |
|---|---|---|---|
| IF (non gélifiée) | Lanol 99 | Isononyl Isononanoate | 99,35 |
| | KF 8004 | Amodimethicone | 0,5 |
| | Phat Black DC 9206 | CI 61565, CI 60725, CI 26100 | 0,15 |
| MF | Lanol 99 | Isononyl Isononanoate | 100 |

Protocole de préparation

Pour l'OF :

- Le carbomère est dispersé dans l'eau osmosée et mis sous agitation pendant 2 heures à l'aide d'une pâle défloculeuse,
- La glycérine, le Zemea ainsi que le butylène glycol sont par la suite ajoutés. Suite à ces ajouts, le mélange est placé sous agitation pendant 10 min,
- Le Microcare PE, Microcare PTG et l'EDETA sont ajoutés. Le mélange est agité pendant 5 min.
- La soude est ensuite ajoutée.
- La dernière étape consiste à mélanger la solution pendant 1 heure.

Pour la base : La soude et l'eau sont mélangées à l'aide d'un barreau magnétique pendant 5 min.

Pour l'IF gélifiée :

- L'amodiméthicone est ajoutée au Lanol 99 puis mélangée à l'aide d'un barreau magnétique pendant 5 min.
- On chauffe le mélange à 80°C puis on ajoute le Rheopearl KL2 sous agitation magnétique.
- Le colorant Phat Black DC 9206 est ensuite ajouté à la composition.
- Ce mélange peut ensuite être placé dans un bain marie chauffé à 75°C sous agitation magnétique pendant 1 heure.

Pour l'IF non gélifiée :

- L'amodiméthicone et le colorant Phat Black DC 9206 sont ajoutés au Lanol 99 puis mélangés à l'aide d'un barreau magnétique pendant 5 min.

Les débits (en mL/h) utilisés pour préparer les compositions 3A et 3B sont les suivants :

| | Composition 3A | Composition 3B |
|---|---|---|
| **OF** | 180 mL/h | 180 mL/h |
| **MF** | - | 5 mL/h |
| **IF** | 20 mL/h | 15 mL/h |
| **Base** | 21,6 mL/h | 21,6 mL/h |

Lors de la fabrication de la composition 3A, l'IF et le dispositif microfluidique sont maintenus à 75°C.

Le pH et la viscosité des compositions 3A (invention) et 3B (comparative) ont été étudiés au cours du temps, à température ambiante et à 50°C. Les résultats sont fournis dans les tableaux suivants :

**pH / composition 3A:**

| T(jour) | **1** | **3** | **7** | **14** | **21** | **28** | **56** | **84** | **98** | **112** | **140** | **154** |
|---|---|---|---|---|---|---|---|---|---|---|---|---|
| ***TA*** | 5,62 | 5,68 | 5,63 | 5,70 | 5,67 | 5,72 | 5,72 | 5,70 | 5,76 | 5,86 | 5,80 | 5,85 |
| ***50°C*** | 5,76 | 5,47 | 5,56 | 5,76 | 5,71 | 5,72 | 5,63 | 5,65 | 5,67 | 5,77 | 5,70 | 5,75 |

**pH / composition 3B:**

| T (jour) | **1** | **3** | **7** | **14** | **21** | **28** | **56** | **84** | **98** | **126** | **140** | **154** |
|---|---|---|---|---|---|---|---|---|---|---|---|---|
| ***TA*** | 5,62 | 5,63 | 5,70 | 5,84 | 5,90 | 5,89 | 5,94 | 5,85 | 5,88 | 5,95 | 5,90 | 5,92 |
| ***50°C*** | 5,52 | 5,72 | 5,77 | 5,79 | 5,84 | 5,82 | 5,75 | 5,70 | 5,74 | 5,82 | 5,83 | 5,80 |

**Viscosité (mPa.s) / composition 3A** (10rpm ; 30 sec) :

| T(jour) | **1** | **3** | **7** | **14** | **21** | **28** | **56** | **84** | **98** | **126** | **140** | **154** |
|---|---|---|---|---|---|---|---|---|---|---|---|---|
| ***TA*** | 10140 | 10360 | 10200 | 10620 | 10560 | 10380 | 10200 | 10000 | 10360 | 10400 | 10500 | 10700 |
| ***50°C*** | 9000 | 8600 | 8760 | 10940 | 11140 | 10000 | 10140 | 10360 | 10080 | 10180 | 10000 | 10050 |

**Viscosité (mPa.s) / composition 3B** (10rpm ; 30 sec) :

| T(jour) | **1** | **3** | **7** | **14** | **21** | **28** | **56** | **84** | **98** | **126** | **140** | **154** |
|---|---|---|---|---|---|---|---|---|---|---|---|---|
| ***TA*** | 10400 | 10300 | 11060 | 10240 | 10040 | 10220 | 10100 | 10040 | 9680 | 9920 | 9950 | 9980 |
| ***50°C*** | 9300 | 7940 | 9580 | 10160 | 9460 | 8920 | 9620 | 9930 | 9780 | 9780 | 9780 | 9760 |

Au vu de ces résultats, il a été montré que la composition 3A selon l'invention, comprenant une phase grasse gélifiée, présente une même évolution du pH et de viscosité que la composition 3B comparative. Les deux compositions se sont avérées avantageusement stables au cours du temps, que ce soit à température ambiante ou à une température de 50°C.

En outre, concernant l'aspect des gouttes, aucune coalescence n'a été observée pour les deux compositions. Ainsi, la présence de l'agent gélifiant dans la composition 3A n'interagit pas avec la phase aqueuse et ne perturbe pas la stabilité du système.

**Exemple 4** : **Préparation d'une composition parfumante**

La composition parfumante de l'exemple 4A (sérum parfum) selon l'invention est un produit parfumé (qui peut être pulvérisé) qui est stable dans le temps.

La composition 4A est une composition dont les gouttes de phase grasse comprennent un agent gélifiant. Elle a été préparée à partir des compositions de phases suivantes (cf. tableau ci-dessous) :

***Composition 4A*** *:*

| **Fluide** | **Nom** | **INCI** | **%w/w** |
|---|---|---|---|
| OF (*μ = 552 mPa.s pH = 4,60*) | Eau Osmosée | - | 95,036 |
| | Glycerol | Glycérine | 1,226 |
| | Microcare PTG | Pentylèneglycol | 2,509 |
| | Microcare PE | Phénoxyéthanol | 0,987 |
| | Carbomer Tego 340 FD | Carbomer | 0,200 |
| | EDETA | Disodium EDTA | 0,040 |
| | NaOH | Hydroxyde de sodium | 0,002 |
| Base | Eau Osmosée | Eau | 99,70 |
| | NaOH | Hydroxyde de sodium | 0,30 |
| IF (gélifiée) | Lanol 99 | Isononyl Isononanoate | 59,50 |
| | **Rheopearl KL2** | Dextrin Palmitate | 10,00 |
| | KF 8004 | Amodimethicone | 0,50 |
| | Technobois | Parfum | 30,00 |

La composition 4B (comparative) est une composition dont les gouttes de phase grasse ne comprennent pas d'agent gélifiant. Elle a été préparée à partir des compositions de phases suivantes (cf. tableau ci-dessous) :

***Composition 4B***

| **Fluide** | **Nom** | **INCI** | **%w/w** |
|---|---|---|---|
| OF (*μ = 552 mPa.s pH = 4,60*) | Eau Osmosée | - | 95,036 |
| | Glycerol | Glycérine | 1,226 |
| | Microcare PTG | Pentylèneglycol | 2,509 |
| | Microcare PE | Phénoxyéthanol | 0,987 |
| | Carbomer Tego 340 FD | Carbomer | 0,200 |
| | EDETA | Disodium EDTA | 0,040 |
| | NaOH | Hydroxyde de sodium | 0,002 |
| Base | Eau Osmosée | Eau | 99,70 |
| | NaOH | Hydroxyde de sodium | 0,30 |
| IF (non gélifiée) | Lanol 99 | Isononyl Isononanoate | 58,50 |
| | KF 8004 | Amodimethicone | 0,50 |
| | Technobois | Parfum | 41,00 |
| MF | Lanol 99 | Isononyl Isononanoate | 100 |

Protocole de préparation

Pour l'OF :

- Le carbomère est tout d'abord dispersé dans l'eau osmosée et mis sous agitation pendant 2 heures à l'aide d'une pâle défloculeuse,
- La glycérine est par la suite ajoutée. Suite à ces ajouts, le mélange est placé sous agitation pendant 10 min,
- Le Microcare PE, Microcare PTG et l'EDETA sont par la suite ajouté. Le mélange est agité pendant 5 min,
- La soude est ensuite ajoutée, et
- La dernière étape consiste à mélanger la solution pendant 1 heure.

Pour la base : La soude et l'eau sont mélangées à l'aide d'un barreau magnétique pendant 5 min.

Pour l'IF gélifiée :

- Le Lanol 99 et le parfum sont tout d'abord mélangés par agitation magnétique (2 min).
- L'amodiméthicone est ajoutée puis mélangée à l'aide d'un barreau magnétique pendant 5 min.
- On chauffe le mélange à 80°C puis on ajoute le Rheopearl KL2 sous agitation magnétique.
- Ce mélange peut ensuite être placé dans un bain marie chauffé à 75°C sous agitation magnétique pendant 1 heure.

Pour l'IF non gélifiée :

- Le Lanol 99 et le parfum sont tout d'abord mélangés par agitation magnétique (2 min).
- L'amodiméthicone et le colorant Phat Black DC 9206 sont ajoutés puis mélangés à l'aide d'un barreau magnétique pendant 5 min.

Les débits (en mL/h) utilisés pour préparer les compositions 4A et 4B sont les suivants :

| | **Composition 4A** | **Composition 4B** |
|---|---|---|
| **OF** | 150 | 150 |
| **MF** | - | 4 |
| **IF** | 16 | 12 |
| **Base** | 10 | 10 |

Lors de la fabrication de la composition 4A, l'IF et le dispositif microfluidique sont maintenus à 75°C.
Le pH et la viscosité des compositions 4A (invention) et 4B (comparative) ont été étudiés au cours du temps, à température ambiante et à 50°C. Les résultats sont fournis dans les tableaux suivants :

**pH / composition 4A** :

| **T**(jours) | **1** | **3** | **7** | **9** | **14** | **21** | **28** |
|---|---|---|---|---|---|---|---|
| ***TA*** | 5,99 | 5,51 | 5,48 | 5,51 | 5,55 | 5,46 | 5,58 |
| ***50°C*** | 4,91 | 5,04 | 5,12 | 5,27 | 5,65 | 5,39 | 5,51 |

**pH / composition 4B** :

| **T**(jours) | **1** | **3** | **7** | **9** | **14** | **21** | **28** |
|---|---|---|---|---|---|---|---|
| ***TA*** | 5,15 | 5,39 | 5,30 | 5,39 | 5,60 | 5,49 | 5,52 |
| ***50°C*** | 5,53 | 5,39 | 5,31 | 5,36 | 5,52 | 5,38 | 5,35 |

**Viscosité (mPa.s) / composition 4A** (10rpm ; 30 sec) :

| **T**(jours) | **1** | **3** | **7** | **9** | **14** | **21** | **28** |
|---|---|---|---|---|---|---|---|
| ***TA*** | 5390 | 3030 | 5850 | 5950 | 5550 | 5380 | 5280 |
| ***50°C*** | 3680 | 3240 | 6450 | 6180 | 6180 | 6390 | 6400 |

**Viscosité (mPa.s) / composition 4B** (10rpm ; 30 sec) :

| **T**(jours) | **1** | **3** | **7** | **9** | **14** | **21** | **28** |
|---|---|---|---|---|---|---|---|
| ***TA*** | 4180 | 2740 | 5330 | 5180 | 5490 | 5350 | 5180 |
| ***50°C*** | 4790 | 3030 | 5850 | 5920 | 5790 | 6140 | 6170 |

Pour les produits parfums 4A et 4B, il a été démontré que les bulles sont réparties de manière homogène dans le gel et aucune coalescence n'est observée durant 3 mois.

En conclusion, les produits avec bulles gélifiées et sans bulles gélifiées ont les mêmes stabilités, pH, viscosités et pouvoirs suspensifs.

**Exemple 5** : **Préparation d'une composition gel-crème avec un colorant**

La composition de l'exemple 5 est un gel-crème. La composition de l'exemple 5 est constituée des ingrédients suivants (cf. tableau ci-dessous) :

| **Nom** | **Nom INCI** | ***% w/w PHASES*** | **% w/w** |
|---|---|---|---|
| **PHASE GEL AQUEUX** | | | |
| Eau osmosée | Eau | QSP* | QSP* |
| Microcare PE | Phénoxyéthanol | 0,96 | 0,86 |
| Microcare Emollient PTG | Pentylèneglycol | 2,36 | 2,12 |
| Tego carbomer 340 FD | Carbomer | 0,32 | 0,29 |
| Aristoflex Velvet | Polyacrylate Crosspolymer-11 | 0,086 | 0,077 |
| Rhodicare T | Xanthane | 0,056 | 0,050 |
| Cellosize Hydroxyethyl cellulose PCG-10 | Hydroxyéthyl Cellulose | 0,027 | 0,024 |
| Glycerine codex (99%) | Glycerin | 5,89 | 5,27 |
| Zemea | Propanediol | 2,99 | 2,68 |
| Butylène Glycol | Butylène Glycol | 2,95 | 2,64 |
| EDETA BD | Disodium EDTA | 0,039 | 0,035 |
| Sodium Hydroxide Pellets PRS codex | Hydroxyde de sodium | 0,049 | 0,044 |
| Total | | *100,00* | *89,62* |
| **PHASE GRASSE** | | | |
| DUB ININ | Isononyl Isononanoate | QSP* | QSP* |
| Rheopearl KL2 | Dextrin Palmitate | 15,00 | 1,56 |
| Creasperse White R | Titanium Dioxide, Hydrogenated Polydecene, Hydroxystearic Acid | 0,051 | 0,0053 |
| Phat Blue DC 6204 | CI 61565 / CI 60725 | 0,0025 | 0,00026 |

(suite)

| PHASE GRASSE | | | |
|---|---|---|---|
| DUB ININ | Isononyl Isononanoate | QSP* | QSP* |
| Nusil CAS 3131 | Amodiméthicone | 0,20 | 0,021 |
| Total | | *100,00* | *10,38* |
| Total | | | 100,00 |
| ** QSP : quantité suffisante pour* | | | |

La composition de l'exemple 5 est préparée selon le protocole suivant :

Outer fluid : OF

| **Nom** | **Nom INCI** | **% w/w** |
|---|---|---|
| Eau osmosée | Aqua | 84,26 |
| Glycerine codex (99%) | Glycerin | 5,89 |
| Zemea | Propanediol | 2,99 |
| Butylène Glycol | Butylene Glycol | 2,95 |
| Microcare emollient PTG | Pentylenglycol | 2,436 |
| Microcare PE | Phenoxyethanol | 0,96 |
| Tego carbomer 340 FD | Carbomer | 0,32 |
| Aristoflex Velvet | Polyacrylate Crosspolymer-11 | 0,086 |
| Rhodicare T | Xanthane | 0,056 |
| Cellosize Hydroxyethyl cellulose PCG-10 | Hydroxyethyl Cellulose | 0,027 |
| EDETA BD | Disodium EDTA | 0,039 |
| Sodium Hydroxide Pellets PRS codex | Hydroxyde de sodium | 0,049 |
| Total | | 100,00 |

Inter fluid : IF

| **Nom** | **Nom INCI** | **% w/w** |
|---|---|---|
| DUB ININ | Isononyl Isononanoate | QSP* |
| Rheopearl KL2 | Dextrin Palmitate | 15,00 |
| Nusil CAS 3131 | Amodimethicone | 0,20 |
| Creasperse White R | Titanium Dioxide, Hydrogenated Polydecene, Hydroxystearic Acid | 0,051 |
| Phat Blue DC 6204 | CI 61565 / CI 60725 | 0,0025 |
| Total | | 100,00 |

Base :

| **Nom** | **Nom INCI** | **% w/w** |
|---|---|---|
| Eau osmosée | Aqua | QSP* |
| NaOH | Sodium Hydroxyde | 2,9929 |

(suite)

| Nom | Nom INCI | % w/w |
|---|---|---|
| Total | | 100,00 |
| * QSP : quantité suffisante pour | | |

**Mode opératoire des phases** :

Pour l'OF :

- Une première phase, appelée OF1, se compose de l'eau et du carbomère. Ce mélange est placé sous agitation à l'aide d'un pâle défloculeuse pendant 2 heures.
- Une deuxième phase, appelée OF 2, est préparée. Elle se compose de la glycérine, du butylène glycol, du Zemea et du Rhodicare T. L'agitation de se mélange se réalise manuellement à l'aide d'une spatule pendant 1min. L'objectif est de dispersée de manière homogène la poudre de Rhodicare T au sein de la phase.
- L'OF 2 est ajoutée, sous agitation, à l'OF1.
- L'aristoflex Velvet est ajouté au mélange à l'aide d'une solution aqueuse concentrée à 1%m en Aristoflex Velvet. De la même manière, le Cellosize hydroxyethyl cellulose PCG-10 est incorporé à l'aide d'une solution aqueuse concentrée en Cellosize hydroxyethyl cellulose PCG-10 à 0,5%m. Une fois ces 2 composés ajoutés, le mélange est agité pendant 1 heure.
- Le Microcare PE, Microcare PTG et l'EDETA sont par la suite ajouté. Le mélange est agité pendant 5 min.
- La soude est ensuite incorporée.
- La dernière étape consiste à mélanger la solution pendant 2 heures.

Pour la base : La soude et l'eau sont mélangées à l'aide d'un barreau magnétique pendant 5 min.

Pour l'IF :

- L'amodiméthicone est ajoutée au DUB ININ puis mélangée à l'aide d'un barreau magnétique pendant 15 min.
- On chauffe le mélange à 80°C puis on ajoute le Rheopearl KL2 sous agitation magnétique.
- Le colorant Phat Black DC 9206 et le Creasperse White R sont ensuite ajoutés, le mélange obtenu étant mélangé à l'aide d'un barreau magnétique pendant 15 min.
- Ce mélange peut ensuite être placé dans un bain marie chauffé à 85°C sous agitation magnétique pendant 1 heure.

Les débits (en mL/h) sont les suivants :

| | |
|---|---|
| **OF** | 150 |
| **MF** | - |
| **IF** | 20 |
| **Base** | 2,475 |

Lors de la fabrication de la composition finale, l'IF et le dispositif microfluidique sont maintenus à 80°C.

La composition finale comprend des gouttes de phase grasse dispersées bleues pâles translucides dans un gel aqueux incolore translucide.

**Exemple 6** : **Préparation d'une composition gel-crème hydratante et anti-âge**

La composition ci-dessous est obtenue à l'aide d'un procédé « non-microfluidique » (i.e. par émulsification) tel que décrit précédemment).

| Nom | Nom INCI | % w/w |
|---|---|---|
| Eau osmosée | Eau | 69,02 |
| Microcare PE | Phénoxyéthanol | 0,86 |
| Microcare Emollient PTG | Pentylèneglycol | 2,12 |
| Tego carbomer 340 FD | Carbomer | 0,29 |
| Aristoflex Velvet | Polyacrylate Crosspolymer-11 | 0,077 |
| Rhodicare T | Xanthane | 0,050 |
| Cellosize Hydroxyethyl cellulose PCG-10 | Hydroxyéthyl Cellulose | 0,024 |
| Glycerine codex (99%) | Glycerin | 5,27 |
| Zemea | Propanediol | 2,68 |
| Butylène Glycol | Butylène Glycol | 2,64 |
| EDETA BD | Disodium EDTA | 0,035 |
| Sodium Hydroxide Pellets PRS codex | Hydroxyde de sodium | 0,044 |
| DUB ININ | Isononyl Isononanoate | 8,35 |
| **Rheopearl KL2** | **Dextrin Palmitate** | 2,01 |
| OriStar RN | Retinol | 0.5 |
| Vitamin E Acetate | Tocopheryl Acetate | 0.5 |
| Ceramide II | Ceramide NG | 0.5 |
| Nikkol VC-IP | Ascorbyl Tetraisopalmitate | 2 |
| Green Tea Phytolait | Camellia Kissi Seed Oil | 3 |
| Creasperse White R | Titanium Dioxide, Hydrogenated Polydecene, Hydroxystearic Acid | 0,0053 |
| Phat Blue DC 6204 | CI 61565 / CI 60725 | 0,00026 |
| Nusil CAS 3131 | Amodiméthicone | 0,021 |
| Total | | 100,00 |

**Exemple 7** : **Préparation d'une composition gel-crème avec un colorant**

La composition de l'exemple 7 est un gel-crème. La composition de l'exemple 7 est constituée des ingrédients suivants (cf. tableau ci-dessous) :

| Nom | Nom INCI | *% w/w phases* | % w/w |
|---|---|---|---|
| **PHASE GEL AQUEUX** | | | |
| Eau osmosée | Eau | QSP* | QSP* |
| Microcare PE | Phénoxyéthanol | 0,96 | 0,86 |
| Microcare Emollient PTG | Pentylèneglycol | 2,36 | 2,12 |
| Tego carbomer 340 FD | Carbomer | 0,32 | 0,29 |
| Aristoflex Velvet | Polyacrylate Crosspolymer-11 | 0,086 | 0,077 |
| Rhodicare T | Xanthane | 0,056 | 0,050 |

(suite)

| Nom | Nom INCI | *% w/w phases* | % w/w |
|---|---|---|---|
| **PHASE GEL AQUEUX** | | | |
| Cellosize Hydroxyethyl cellulose PCG-10 | Hydroxyéthyl Cellulose | 0,027 | 0,024 |
| Glycerine codex (99%) | Glycerin | 5,89 | 5,27 |
| Zemea | Propanediol | 2,99 | 2,68 |
| Butylène Glycol | Butylène Glycol | 2,95 | 2,64 |
| EDETA BD | Disodium EDTA | 0,039 | 0,035 |
| Sodium Hydroxide Pellets PRS codex | Hydroxyde de sodium | 0,049 | 0,044 |
| Total | | *100,00* | *89,62* |
| **PHASE GRASSE** | | | |
| DUB ININ | Isononyl Isononanoate | QSP* | QSP* |
| **Rheopearl MKL2** | **Dextrin Myristate** | 15 | 2,01 |
| Creasperse White R | Titanium Dioxide, Hydrogenated Polydecene, Hydroxystearic Acid | 0,051 | 0,0053 |
| Phat Blue DC 6204 | CI 61565 / CI 60725 | 0,0025 | 0,00026 |
| Nusil CAS 3131 | Amodiméthicone | 0,20 | 0,021 |
| Total | | *100,00* | *10,38* |
| **Total** | | | **100,00** |
| ** QSP : quantité suffisante pour* | | | |

La composition de l'exemple 7 est préparée selon un protocole identique à celui décrit en exemple 5, à la différence que :

- l'agent gélifiant Rheopearl KL2 est remplacé par le Rheopearl MKL2,
- le chauffage pour la préparation de l'IF est de 90°C, et
- lors de la fabrication de la composition finale, l'IF et le dispositif microfluidique sont maintenus à 85°C.

Les débits (en mL/h) sont identiques à ceux présentés en exemple 5.

La composition finale comprend des gouttes de phase grasse dispersées bleues pales translucides dans un gel aqueux incolore translucide. La phase grasse dispersée de la composition de l'exemple 7 a une transparence améliorée par rapport à celle de la composition de l'exemple 5. L'agent gélifiant Rheopearl MKL2 a donc un effet avantageux en termes de transparence de la phase grasse dispersée d'une composition selon l'invention.

**Exemple 8** : **Préparation d'une composition gel-crème**

La composition de l'exemple 8 est un gel-crème. La composition de l'exemple 8 est constituée des ingrédients suivants (cf. tableau ci-dessous) :

| Nom | Nom INCI | *% w/w PHASES* | % w/w |
|---|---|---|---|
| **PHASE GEL AQUEUX** | | | |
| Eau osmosée | Eau | QSP* | QSP* |
| Microcare PE | Phénoxyéthanol | 0,96 | 0,86 |
| Microcare Emollient PTG | Pentylèneglycol | 2,36 | 2,12 |

(suite)

| Nom | Nom INCI | % *w/w PHASES* | % w/w |
|---|---|---|---|
| **PHASE GEL AQUEUX** | | | |
| Tego carbomer 340 FD | Carbomer | 0,32 | 0,29 |
| Rhodicare T | Xanthane | 0,056 | 0,050 |
| Glycerine codex (99%) | Glycerin | 5,89 | 5,27 |
| Zemea | Propanediol | 2,99 | 2,68 |
| Butylène Glycol | Butylène Glycol | 2,95 | 2,64 |
| EDETA BD | Disodium EDTA | 0,039 | 0,035 |
| Sodium Hydroxide Pellets PRS codex | Hydroxyde de sodium | 0,049 | 0,044 |
| Total | | *100,00* | *89,62* |
| **PHASE GRASSE** | | | |
| DUB ININ | Isononyl Isononanoate | QSP* | QSP* |
| **Rheopearl KL2** | **Dextrin Palmitate** | **10,00** | **1,038** |
| **Rheopearl MKL2** | **Dextrin Myristate** | **5,00** | **0,519** |
| Nusil CAS 3131 | Amodiméthicone | 0,20 | 0,021 |
| Total | *100,00* | *100* | 10,38 |
| Total | 100,00 | | |
| ** QSP : quantité suffisante pour* | | | |

La composition de l'exemple 8 est préparée selon un protocole identique à celui décrit en exemple 5, à la différence que :

- l'IF est dénuée de colorant et l'IF comprend en outre du Rheopearl MKL2 (ajout dans l'IF réalisé simultanément à l'ajout du Rheopearl KL2), et
- la préparation de l'IF est réalisée à 90°C.

Les débits (en mL/h) sont identiques à ceux présentés en exemple 5.

La composition finale comprend des gouttes de phase grasse dispersées translucides dans un gel aqueux incolore translucide. Le mélange d'agents gélifiants « Rheopearl KL2 / Rheopearl MKL2 » est avantageux par rapport au Rheopearl KL2 seul en ce qu'il permet d'obtenir une phase grasse d'une transparence améliorée.

**Exemple 9 : Préparation d'une composition gel-crème**

La composition de l'exemple 9 est un gel-crème. La composition de l'exemple 9 est constituée des ingrédients suivants (cf. tableau ci-dessous) :

| Nom | Nom INCI | % *w/w PHASES* | % w/w |
|---|---|---|---|
| **PHASE GEL AQUEUX** | | | |
| Eau osmosée | Eau | QSP* | QSP* |
| Microcare PE | Phénoxyéthanol | 0,96 | 0,86 |
| Microcare Emollient PTG | Pentylèneglycol | 2,36 | 2,12 |
| Tego carbomer 340 FD | Carbomer | 0,32 | 0,29 |
| Rhodicare T | Xanthane | 0,056 | 0,050 |

(suite)

| Nom | Nom INCI | *% w/w PHASES* | % w/w |
|---|---|---|---|
| **PHASE GEL AQUEUX** | | | |
| Glycerine codex (99%) | Glycerin | 5,89 | 5,27 |
| Zemea | Propanediol | 2,99 | 2,68 |
| Butylène Glycol | Butylène Glycol | 2,95 | 2,64 |
| EDETA BD | Disodium EDTA | 0,039 | 0,035 |
| Sodium Hydroxide Pellets PRS codex | Hydroxyde de sodium | 0,049 | 0,044 |
| Total | | *100,00* | *89,62* |
| **PHASE GRASSE** | | | |
| DUB ININ | Isononyl Isononanoate | 5 | 0,52 |
| Myritol 318 | Caprylic/Capric Triglyceride | 92,35 | 9,58 |
| Aerosil R202 | silica dimethicone silylate | 2,5 | 0,26 |
| Nusil CAS 3131 | Amodiméthicone | 0,15 | 0,015 |
| Total | *100,00* | *100* | *10,38* |
| **Total** | **100,00** | | |

La composition de l'exemple 9 est préparée selon un protocole identique à celui décrit en exemple 5, à la différence que :

- pour l'IF :
  - l'amodiméthicone est ajoutée au DUB ININ puis mélangée à l'aide d'un barreau magnétique pendant 15 min,
  - le Myritol 318 est ajouté au mélange à l'aide d'une agitation magnétique,
  - le mélange est alors placé sous agitation mécanique à l'aide d'une pâle défloculeuse, et
  - l'Aerosil R202 est ensuite ajouté au mélange sous agitation, maintenue pendant 20 min.
- toutes les étapes de préparations des phases, en particulier l'IF, de même que le procédé de fabrication de la composition sont réalisées à température ambiante.

Les débits (en mL/h) sont identiques à ceux présentés en exemple 5.

La composition finale comprend des gouttes de phase grasse dispersées dans un gel aqueux incolore translucide. L'agent gélifiant Aerosil R202 est avantageux en ce qu'il confère à la phase grasse un comportement thixotrope ce qui autorise la fabrication d'une dispersion selon l'invention par mise en œuvre d'un procédé microfluidique dont toutes les étapes sont réalisées à température ambiante.

## Revendications

1. Dispersion contenant une phase dispersée comprenant des gouttes et une phase aqueuse continue, de préférence sous forme de gel, dans laquelle les gouttes comprennent une phase grasse contenant au moins un agent gélifiant et une écorce, ladite écorce comprenant au moins un polymère anionique et au moins un polymère cationique, ladite dispersion étant différente d'une composition comprenant : 32,312% en poids d'eau, 0,8% en poids de phénoxyéthanol, 2% en poids de pentylèneglycol, 0,25% en poids de carbomère, 24,57% en poids de glycérine, 0,03% en poids d'EDTA disodique, 0,038% en poids d'hydroxyde de sodium, 12,8% en poids de polymère dimethicone/vinyl dimethicone crosspolymer, 27% en poids de diméthicone et 0,2% en poids d'amodiméthicone par rapport au poids total de ladite composition.

2. Dispersion selon la revendication 1, dans laquelle l'agent gélifiant est choisi parmi les agents gélifiants lipophiles organiques ou minéraux, polymériques ou moléculaires ; les corps gras solides à température et pression ambiante ;

et leurs mélanges.

3. Dispersion selon la revendication 2, dans laquelle l'agent gélifiant est choisi dans le groupe constitué des polyacrylates, des esters de dextrine et d'acide(s) gras, des esters de glycérol et d'acide(s) gras, des polyamides, et de leurs mélanges, et est de préférence choisi parmi les esters de dextrine et d'acide(s) gras, et mieux les esters de dextrine et d'acide(s) gras choisis dans le groupe constitué des palmitates de dextrine, des myristates de dextrine, des palmitates/éthylhexanoates de dextrine, et de leurs mélanges.

4. Dispersion selon la revendication 2, dans laquelle les agents gélifiants sont choisis dans le groupe constitué des argiles éventuellement modifiées, des silices éventuellement traitées hydrophobes, telles que la silice pyrogénée, et de leurs mélanges.

5. Dispersion selon l'une quelconque des revendications 1 à 4, comprenant de 0,5% à 99,99%, de préférence de 1% à 70%, en particulier de 1,5% à 50%, mieux de 2% à 40%, en particulier de 2,5% à 30%, et préférentiellement de 10 % à 20%, en poids d'agent(s) gélifiant(s) par rapport au poids total de la phase grasse.

6. Dispersion selon l'une quelconque des revendications 1 à 5, dans laquelle le polymère cationique est un polymère silicone modifié par une fonction amine primaire, secondaire ou tertiaire, tel que l'amodiméthicone, de préférence ledit polymère cationique répond à la formule suivante :

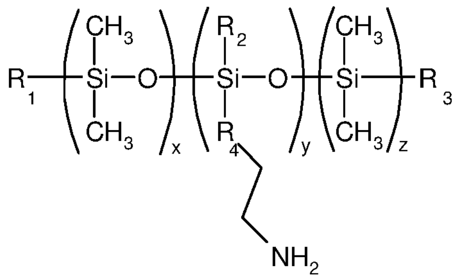

dans laquelle :

- $R_1$, $R_2$ et $R_3$, indépendamment les uns des autres, représentent OH ou $CH_3$ ;
- $R_4$ représente un groupe $-CH_2-$ ou un groupe -X-NH- dans lequel X est un radical alkylène divalent en C3 ou C4 ;
- x est un nombre entier compris entre 10 et 5 000 ;
- y est un nombre entier compris entre 2 et 1 000 ; et
- z est un nombre entier compris entre 0 et 10.

7. Dispersion selon l'une quelconque des revendications 1 à 6, dans laquelle chaque goutte comprend de 0,01% à 10%, de préférence de 0,05% à 5%, en poids de polymère(s) cationique(s), notamment d'amodiméthicone(s), par rapport au poids total de la phase grasse.

8. Dispersion selon l'une quelconque des revendications 1 à 7, dans laquelle le polymère anionique est un polymère comprenant des unités monomères comportant au moins une fonction chimique acide carboxylique.

9. Dispersion selon l'une quelconque des revendications 1 à 8, dans laquelle ladite dispersion comprend de 0,01% à 5%, de préférence de 0,05% à 2%, et préférentiellement de 0,10% à 0,5%, en poids de polymère(s) anionique(s), notamment de carbomère(s), par rapport au poids total de ladite dispersion.

10. Dispersion selon l'une quelconque des revendications 1 à 9, comprenant en outre au moins un actif biologique/cosmétique choisi parmi les agents hydratants, les agents cicatrisants, les agents dépigmentants, les filtres UV, les agents desquamants, les agents antioxydants, les actifs stimulant la synthèse des macromoléculaires dermiques et/ou épidermiques, les agents dermodécontractants, les agents anti-transpirants, les agents apaisants et/ou les agents anti-âge, et leurs mélanges.

11. Dispersion selon l'une quelconque des revendications 1 à 10, dans laquelle le diamètre moyen des gouttes de la phase dispersée est compris de 0,2 μm à 3 000 μm, de préférence de 20 μm à 2 500 μm, et préférentiellement de 500 μm à 1 500 μm.

**12.** Dispersion selon l'une quelconque des revendications 1 à 11, **caractérisée en ce qu'**elle ne comprend pas de tensioactif.

**13.** Dispersion selon l'une quelconque des revendications 1 à 12, comprenant au moins 5% en poids de glycérine par rapport au poids total de ladite dispersion.

**14.** Procédé de préparation de la dispersion telle que définie selon l'une quelconque des revendications 1 à 13, comprenant les étapes suivantes :

- optionnellement, le chauffage d'un fluide huileux FI, à une température comprise de 40°C à 150°C ;
- la mise en contact d'un fluide aqueux FE et du fluide huileux FI ; et
- la formation des gouttes de phase grasse, constituée du fluide huileux FI, dispersée dans une phase aqueuse continue, constituée de fluide FE, lesdites gouttes comprenant une écorce isolant le cœur des gouttes de la phase grasse de la dispersion,

dans lequel :

- le fluide huileux FI comprend au moins un polymère cationique, en particulier l'amodiméthicone, et au moins un agent gélifiant, et
- le fluide aqueux FE comprend au moins de l'eau et au moins un polymère anionique, en particulier un carbomère.

**15.** Procédé de préparation d'une composition, notamment cosmétique, comprenant l'association d'une dispersion selon l'une quelconque des revendications 1 à 13, avec un milieu physiologiquement acceptable.

**16.** Procédé non thérapeutique de traitement cosmétique d'une matière kératinique, notamment la peau et/ou les cheveux, et plus particulièrement la peau, comprenant une étape d'application sur ladite matière kératinique d'une dispersion selon l'une quelconque des revendications 1 à 13 ou d'au moins une couche d'une composition cosmétique préparée selon le procédé de la revendication 15.

**17.** Utilisation d'au moins un agent gélifiant de phase grasse pour améliorer la résistance mécanique de gouttes d'une dispersion contenant une phase aqueuse continue, de préférence sous forme de gel, et une phase grasse dispersée comprenant les gouttes, ladite dispersion étant telle que définie selon l'une quelconque des revendications 1 à 13.

## Patentansprüche

**1.** Dispersion, die eine dispergierte Phase, die Tropfen umfasst, und eine kontinuierliche wässrige Phase, vorzugsweise in Gelform, enthält, wobei die Tropfen eine Fettphase, die mindestens ein Geliermittel enthält, und eine Schale umfassen, wobei die Schale mindestens ein anionisches Polymer und mindestens ein kationisches Polymer umfasst, wobei die Dispersion von einer Zusammensetzung verschieden ist, die umfasst: 32,312 Gew.-% Wasser, 0,8 Gew.-% Phenoxyethanol, 2 Gew.-% Pentylenglykol, 0,25 Gew.-% Carbomer, 24,57 Gew.-% Glycerin, 0,03 Gew.-% Dinatrium-EDTA, 0,038 Gew.-% Natriumhydroxid, 12,8 Gew.-% Dimethicon/Vinyldimethicon-Crosspolymer, 27 Gew.-% Dimethicon und 0,2 Gew.-% Amodimethicon, bezogen auf das Gesamtgewicht der genannten Zusammensetzung.

**2.** Dispersion nach Anspruch 1, wobei das Geliermittel ausgewählt ist aus organischen oder anorganischen, polymeren oder molekularen lipophilen Geliermitteln; festen Fetten bei Umgebungstemperatur und -druck; und Mischungen davon.

**3.** Dispersion nach Anspruch 2, wobei das Geliermittel aus der Gruppe ausgewählt ist, die aus Polyacrylaten, Dextrinfettsäureestern, Glycerinfettsäureestern, Polyamiden und Mischungen davon besteht, und vorzugsweise aus Dextrinfettsäureestern und besonders bevorzugt aus Dextrinfettsäureestern ausgewählt ist, die aus der Gruppe ausgewählt sind, die aus Dextrinpalmitaten, Dextrinmyristaten, Dextrinpalmitaten/Ethylhexanoaten und Mischungen davon besteht.

**4.** Dispersion nach Anspruch 2, wobei die Geliermittel ausgewählt sind aus der Gruppe bestehend aus gegebenenfalls modifizierten Tonen, gegebenenfalls hydrophob behandelten Kieselsäuren, wie pyrogener Kieselsäure, und Mischungen davon.

**5.** Dispersion nach einem beliebigen der Ansprüche 1 bis 4, umfassend 0,5 % bis 99,99 %, vorzugsweise 1 % bis 70 %, insbesondere 1,5 % bis 50 %, noch bevorzugter 2 % bis 40 %, insbesondere 2,5 % bis 30 % und vorzugsweise 10 % bis 20 % an Geliermittel(n) bezogen auf das Gesamtgewicht der Fettphase.

**6.** Dispersion nach einem beliebigen der Ansprüche 1 bis 5, bei der das kationische Polymer ein durch eine primäre, sekundäre oder tertiäre Aminfunktion modifiziertes Silikonpolymer, wie Amodimethicon, ist, wobei das kationische Polymer vorzugsweise der folgenden Formel entspricht:

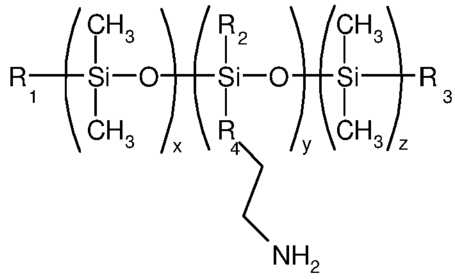

in dem:

- $R_1$, $R_2$ und $R_3$ stehen unabhängig voneinander für OH oder $CH_3$;
- $R_4$ eine $-CH_2-$ Gruppe oder eine -X-NH- Gruppe darstellt, in der X ein zweiwertiger $C_3$- oder $C_4$ Alkylenrest ist;
- x ist eine ganze Zahl zwischen 10 und 5000;
- y ist eine ganze Zahl zwischen 2 und 1000; und
- z ist eine ganze Zahl zwischen 0 und 10.

**7.** Dispersion nach einem beliebigen der Ansprüche 1 bis 6, bei der jeder Tropfen 0,01 bis 10 Gew.-%, vorzugsweise 0,05 bis 5 Gew.-%, kationische(s) Polymer(e), insbesondere Amodimethicon(e), bezogen auf das Gesamtgewicht der Fettphase enthält.

**8.** Dispersion nach einem beliebigen der Ansprüche 1 bis 7, wobei das anionische Polymer ein Polymer ist, das Monomereinheiten mit mindestens einer chemischen Carbonsäurefunktion umfasst.

**9.** Dispersion nach einem beliebigen der Ansprüche 1 bis 8, wobei die Dispersion 0,01 Gew.-% bis 5 Gew.-%, vorzugsweise 0,05 Gew.-% bis 2 Gew.-% und vorzugsweise 0,10 Gew.-% bis 0,5 Gew.-% anionische(s) Polymer(e), insbesondere Carbomer(e), bezogen auf das Gesamtgewicht der Dispersion enthält.

**10.** Dispersion nach einem beliebigen der Ansprüche 1 bis 9, ferner umfassend mindestens einen biologischen/kosmetischen Wirkstoff, ausgewählt aus feuchtigkeitsspendenden Mitteln, heilenden Mitteln, depigmentierenden Mitteln, UV-Filtern, abschuppenden Mitteln, antioxidativen Mitteln, Wirkstoffen, die die Synthese von dermalen und/oder epidermalen Makromolekülen stimulieren, dermodekontrahierenden Mitteln, schweißhemmenden Mitteln, beruhigenden Mitteln und/oder Anti-Aging-Mitteln und Mischungen davon.

**11.** Dispersion nach einem beliebigen der Ansprüche 1 bis 10, wobei der mittlere Tröpfchendurchmesser der dispergierten Phase von 0,2 μm bis 3000 μm, vorzugsweise von 20 μm bis 2500 μm, und vorzugsweise von 500 μm bis 1500 μm beträgt.

**12.** Dispersion nach einem beliebigen der Ansprüche 1 bis 11, **dadurch gekennzeichnet, dass** sie kein Tensid enthält.

**13.** Dispersion nach einem beliebigen der Ansprüche 1 bis 12, umfassend mindestens 5 Gew.-% Glycerin, bezogen auf das Gesamtgewicht der Dispersion.

**14.** Verfahren zur Herstellung der Dispersion, wie nach einem beliebigen der Ansprüche 1 bis 13 definiert, die folgenden Schritte umfassend:

- optional, Erhitzen einer Öl-Flüssigkeit FI auf eine Temperatur von 40°C bis 150°C;
- Inkontaktbringen einer wässrigen Flüssigkeit FE und der Öl-Flüssigkeit FI; und
- Bilden von Tropfen der Fettphase, die von der Öl-Flüssigkeit FI gebildet wird, dispergiert in einer kontinuierlichen

wässrigen Phase, die von der Flüssigkeit FE gebildet wird, wobei die Tropfen eine Schale umfassen, die den Kern der Tropfen von der Fettphase der Dispersion isoliert,
bei dem:

- die Öl-Flüssigkeit FI mindestens ein kationisches Polymer, insbesondere Amodimethicon, und mindestens ein Geliermittel enthält, und
- die wässrige Flüssigkeit FE mindestens Wasser und mindestens ein anionisches Polymer, insbesondere ein Carbomer, enthält.

**15.** Verfahren zur Herstellung einer Zusammensetzung, insbesondere einer kosmetischen Zusammensetzung, umfassend die Kombination einer Dispersion nach einem beliebigen der Ansprüche 1 bis 13 mit einem physiologisch akzeptablen Medium.

**16.** Nicht-therapeutisches Verfahren zur kosmetischen Behandlung eines keratinischen Materials, insbesondere der Haut und/oder der Haare, und insbesondere der Haut, umfassend einen Schritt des Auftragens einer Dispersion nach einem beliebigen der Ansprüche 1 bis 13 oder mindestens einer Schicht einer kosmetischen Zusammensetzung, die nach dem Verfahren des Anspruchs 15 hergestellt wurde, auf das keratinische Material.

**17.** Verwendung mindestens eines Fettphasengeliermittels zur Verbesserung der mechanischen Festigkeit von Tropfen einer Dispersion, die eine kontinuierliche wäßrige Phase, vorzugsweise in Gelform, und eine die Tropfen umfassende dispergierte Fettphase enthält, wobei die Dispersion wie in einem beliebigen der Ansprüche 1 bis 13 definiert ist.

## Claims

**1.** Dispersion containing a dispersed phase comprising drops and a continuous aqueous phase, preferably in the form of a gel, in which the drops comprise a fatty phase containing at least one gelling agent and a shell, wherein the shell comprises at least one anionic polymer and at least one cationic polymer, said dispersion being different from a composition comprising: 32.312% by weight of water, 0.8% by weight of phenoxyethanol, 2% by weight of pentyleneglycol, 0.25% by weight of carbomer, 24.57% by weight of glycerin, 0.03% by weight of disodium EDTA, 0.038% by weight of sodium hydroxide, 12.8% by weight of dimethicone (and) dimethicone/vinyl dimethicone crosspolymer, 27% by weight of dimethicone and 0.2% by weight of amodimethicone in relation to the total weight of the composition.

**2.** Dispersion according to claim 1, wherein the gelling agent is chosen from organic or inorganic, polymeric or molecular lipophilic gelling agents; solid fatty substances at ambient temperature and pressure; and their mixtures.

**3.** Dispersion according to claim 2, wherein the gelling agent is selected from the group consisting of polyacrylates, esters of dextrin and fatty acid(s), glycerol esters of fatty acid(s), polyamides, and mixtures thereof, and is preferably selected from the esters of dextrin and fatty acid(s), and even more preferably selected from the esters of dextrin and fatty acid(s) selected from the group consisting of dextrin palmitates, dextrin myristates, dextrin palmitates/ethylhexanoates, and mixtures thereof.

**4.** Dispersion according to claim 2, wherein the gelling agents are selected from the group consisting of optionally modified clays, silicas optionally treated hydrophobic, such as fumed silica, and mixtures thereof.

**5.** Dispersion according to any one of the claims 1 to 5, comprising from 0.5% to 99.99%, preferably from 1% to 70%, in particular from 1.5% to 50%, better from 2% to 40%, in particular from 2.5% to 30%, and preferably from 10% to 20%, by weight of gelling agent(s) relative to the total weight of the fatty phase.

**6.** Dispersion according to any one of the claims 1 to 6, wherein the cationic polymer is a silicone polymer modified with a primary, secondary or tertiary amine function, such as amodimethicone, and preferably said cationic polymer has the following formula:

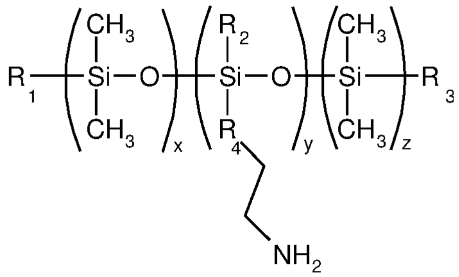

in which:

- $R_1$, $R_2$ and $R_3$, independently of each other, represent OH or $CH_3$;
- $R_4$ represents a group $-CH_2-$ or a group -X-NH-, in which X is a divalent alkylene radical in $C_3$ or $C_4$;
- x is an integer from 10 to 5000;
- y is an integer between 2 and 1000; and
- z is an integer between 0 and 10.

**7.** Dispersion according to any one of the claims 1 to 6, wherein each drop comprises from 0.01% to 10%, preferably from 0.05% to 5%, by weight of cationic polymer(s), in particular amodimethicone(s), relative to the total weight of the fatty phase.

**8.** Dispersion according to any one of the claims 1 to 7, wherein the anionic polymer is a polymer comprising monomeric units having at least one carboxylic acid chemical function.

**9.** Dispersion according to any one of the claims 1 to 8, wherein the dispersion comprises from 0.01% to 5%, preferably from 0.05% to 2%, and preferably from 0.10% to 0.5%, by weight of anionic polymer(s), in particular of carbomer(s), relative to the total weight of the dispersion.

**10.** Dispersion according to any one of the claims 1 to 9, further comprising at least one biological/cosmetic active agent chosen from hydrating agents, cicatrizing agents, depigmenting agents, UV filters, desquamating agents, antioxidants, active agents stimulating the synthesis of dermal and/or epidermal macromoleculars, dermodecontracting agents, antiperspirants, soothing agents and/or anti-aging agents, and mixtures thereof.

**11.** Dispersion according to any one of the claims 1 to 10, wherein the average diameter of the drops of the dispersed phase is from 0.2 μm to 3,000 μm, preferably from 20 μm to 2,500 μm, and preferably from 500 μm to 1,500 μm.

**12.** Dispersion according to any one of the claims 1 to 11, **characterized in that** it does not comprise surfactant.

**13.** Dispersion according to any one of the claims 1 to 12, comprising at least 5% by weight of glycerin relative to the total weight of the dispersion.

**14.** Method for preparing the dispersion according to any one of the claims 1 to 13, comprising the following steps:

- optionally heating an oily fluid FI at a temperature of from 40°C to 150°C;
- contacting an aqueous fluid FE and the oily fluid FI; and
- the formation of drops of fatty phase, consisting of the oily fluid FI, dispersed in a continuous aqueous phase, constituted by fluid FE, wherein the drops comprise a shell isolating the core of the drops of the fatty phase of the dispersion,

in which:

- the oily fluid FI comprises at least one cationic polymer, in particular amodimethicone, and at least one gelling agent, and
- the aqueous fluid FE comprises at least water and at least one anionic polymer, in particular a carbomer.

**15.** Composition, in particular a cosmetic composition, comprising a dispersion according to any one of claims 1 to 13, in association with a physiologically acceptable medium.

**16.** Non-therapeutic method for the cosmetic treatment of a keratin material, in particular the skin and/or the hair, and more particularly the skin, comprising a step of applying to the keratin material a dispersion according to any one of the claims 1 to 13 or at least one layer of a cosmetic composition prepared according to the process of claim 15.

**17.** Use of at least one fatty phase gelling agent for improving the mechanical resistance of drops of a dispersion containing a continuous aqueous phase, preferably in the form of a gel, and a dispersed fatty phase comprising the drops, wherein the dispersion is as defined in any one of claims 1 to 13.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2012120043 A
- FR 2972367
- FR 2976824
- US 20060141046 A
- WO 2010063937 A
- WO 02056847 A
- WO 0247619 A
- US 5783657 A
- US 5874069 A
- US 5919441 A
- US 6051216 A
- US 5981680 A
- FR 2792190 A
- JP 2295912 A
- EP 2353577 A
- WO 2015055748 A


### Littérature non-brevet citée dans la description


- **SATO et al.** *J. Chem. Phys.,* 2007, vol. 111, 1393-1401
- **UTADA et al.** *MRS Bulletin,* 2007, vol. 32, 702-708
- **CRAMER et al.** *Chem. Eng. Sci.,* 2004, vol. 59 (15), 3045-3058